(19) Europäisches Patentamt
European Patent Office
Office européen des brevets

(11) Veröffentlichungsnummer: **0 117 476**
**B1**

(12) # EUROPÄISCHE PATENTSCHRIFT

(45) Veröffentlichungstag der Patentschrift: **16.09.87**

(21) Anmeldenummer: **84101456.6**

(22) Anmeldetag: **13.02.84**

(51) Int. Cl.⁴: **C 07 D 309/04**, C 07 D 309/06, C 07 D 309/10, C 07 D 309/12, C 07 D 309/30, C 07 D 405/04, C 07 D 405/06, C 07 D 407/04, C 07 D 407/06, C 07 D 405/12, C 07 D 407/12

(54) **Tetrahydropyrane.**

(30) Priorität: **28.02.83 DE 3306960**

(43) Veröffentlichungstag der Anmeldung: **05.09.84 Patentblatt 84/36**

(45) Bekanntmachung des Hinweises auf die Patenterteilung: **16.09.87 Patentblatt 87/38**

(84) Benannte Vertragsstaaten: **CH DE FR GB LI**

(56) Entgegenhaltungen:
**CHEMICAL ABSTRACTS, vol. 96, no. 15, 12. April 1982, Columbus, Ohio, USA, DANHEISER Rick L., MORIN John M. Jr., YU Melvin, BASAK Ajoy "Cationic cyclizations initiated by electrocyclic cleavage of cyclopropanes. Synthesis of lactones, tetrahydropyrans, and tetrahydrofurans", Seite 677, Spalte 2, Zusammenfassung No. 122 569v**
**CHEMICAL ABSTRACTS, vol. 95, no. 11, 14. September 1981, Columbus, Ohio, USA, CORBIN Dale H, HOBBS Gregory D, WOODYARD James D. "Stereochemical assignments of the 7-phenyl-3-oxabicyclo [4.1.0] heptanes", Seite 573, Spalte 1, Zusammenfassung No. 96 858w**
**CHEMICAL ABSTRACTS, vol. 93, no. 25, 22. Dezember 1980, Columbus, Ohio, USA, JAKOVAC Ignac J, NG George, LOK Kar P., JONES J. Bryan "Preparation of useful chiral lactone synthons via stereospecific enzyme-catalyzed oxidations of meso-diols", Seite 800, Spalte 1, Zusammenfassung No. 238 869k**
**CHEMICAL ABSTRACTS, vol. 91, no. 3, 16. Juli 1979, Columbus, Ohio, USA, FICINI J., KAHN Ph., FALOU S., TOUZIN A.M., "Generation and synthetic use of 2,3-dihydropyran-3-yl lithium", Seite 630, Spalte 2, Zusammenfassung No. 20 243p**

(73) Patentinhaber: **Merck Patent Gesellschaft mit beschränkter Haftung, Frankfurter Strasse 250, D-6100 Darmstadt (DE)**

(72) Erfinder: **Eidenschink, Rudolf, Dr., Erlenweg 17, D-6110 Dieburg (DE)**
Erfinder: **Krause, Joachim, Dr., Samuel-Morse-Strasse 14, D-6110 Dieburg (DE)**
Erfinder: **Fuss, Peter, Dr., Im Seegraben 5, D-6109 Mühltal-Traisa (DE)**

(56) Entgegenhaltungen: (Fortsetzung)
**CHEMICAL ABSTRACTS, vol. 90, no. 23, 4. June 1979, Columbus, Ohio, USA, HAENSEL R., SCHULZ J., "Total synthesis of 5-acetoxy-6-methoxykawain", Seite 642, Spalte 1, Zusammenfassung No. 186 721x**
**CHEMICAL ABSTRACTS, vol. 89, no. 13, 25. September 1978, Columbus, Ohio, USA, MIHAILOVIC M.L., CEKOVIC Z., STANKOVIC J., DJOKIC-MAZINJANIN S., MARINKOVIC D., KONSTANTINOVIC St., "Reactions with lead tetraacetate. 37. The formation of cyclic ethers from olefinic alcohols. IV. The oxidative cyclization of 4-penten-1-o1 by means of tetravalent lead compounds", Seite 866, Spalte 1, Zusammenfassung No.108 939a**
**CHEMICAL ABSTRACTS, vol. 86, no. 1, 3. Januar 1977, Columbus, Ohio, USA, KUWAJIMA I., MINAMI N., SATO T., "A conveniant method for the preparation of beta-hydroxy esters", Seite 423, Spalte 2, Zusammenfassung No. 4 902u**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 117 476 B1

ACTORUM AG

**Beschreibung**

Die Erfindung betrifft neue Tetrahydropyrane der Formel I

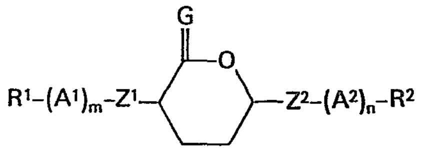

I

worin

G $H_2$ oder =O,

$R^1$ und $R^2$ jeweils eine Alkylgruppe mit 1–10 C-Atomen, worin auch eine oder zwei $CH_2$-Gruppen durch O-Atome ersetzt sein können, F, Cl, Br oder CN,

$R^2$ auch H,

$A^1$ und $A^2$ jeweils unsubstituierte oder durch 1–4 F-Atome substituierte 1,4-Phenylen-, 1,4-Cyclohexylen-, 1,3-Dioxan-2,5-diyl-, Piperidin-1,4-diyl-, 1,4-Bicyclo(2,2,2)-octylen- oder Pyrimidin-2,5-diylgruppen,

$Z^1$ und $Z^2$ jeweils $-CO-O-$, $-O-CO-$, $-CH_2CH_2-$, $-OCH_2-$, $-CH_2O-$ oder eine Einfachbindung,

m und n jeweils 0, 1, 2 oder 3 bedeuten,

(m + n) jedoch mindestens 1 und höchstens 3 sind,

wobei für m = 2 oder 3 die Gruppen $A^1$ und für n = 2 oder 3 die Gruppen $A^2$ jeweils gleich oder voneinander verschieden sein können,

sowie die Säureadditionssalze der basischen unter diesen Verbindungen.

Der Einfachheit halber bedeuten im folgenden «A» die 2-G-tetrahydropyran-3,6-diylgruppe, «Phe» eine 1,4-Phenylgruppe, «Cy» eine 1,4-Cyclohexylengruppe, «Dio» eine 1,3-Dioxan-2,5-diylgruppe, «Bi» eine Bicyclo-(2,2,2)-octylengruppe, «Pip» eine Piperidin-1,4-diylgruppe, und «Pyr» eine Pyrimidin-2,5-diylgruppe, wobei diese Gruppen, insbesondere die 1,4-Phenylengruppe, unsubstituiert oder (bis auf A) durch 1–4 Fluoratome substituiert sein können.

Ähnliche Verbindungen sind z.B. aus der EP-A 19 665 bekannt. Die dort angegebenen Verbindungen enthalten jedoch im Gegensatz zu den vorliegenden keine Tetrahydropyranringe. Weiterhin ist in der Dissertation B. Kohne, Technische Universität Berlin, 1981, Seite 83, eine allgemeine Formel als «Strukturvorschlag» angegeben, die der Formel I [trans-Form; G = $H_2$, $Z^1$ und $Z^2$ = Einfachbindungen, $R^1-(A^1)_m-$ = «z.B. 4-Cyanophenyl-, 4-Alkylphenyl-, 4-Alkylcyclohexyl-», $-(A^2)_n-R^2$ = OR, worin R «z.B. Alkyl ist»] entspricht. Ferner sind Tetrahydropyranderivate beschrieben in Chemical Abstracts, 1982, 96 (15), 122569 v; 1981, 95(11), 96858w; 1980, 93(25), 238 869 k; 1979, 91(3), 20243p; 1979, 90(23), 186721x; 1978, 89(13), 108939a; 1977, 86(1), 4902u; es ist dort jedoch keine Verwendung dieser Substanzen als Komponenten flüssigkristalliner Dielektrika angegeben.

Die Verbindungen der Formel I können wie ähnliche Verbindungen als Komponenten flüssigkristalliner Dielektrika verwendet werden, insbesondere für Displays, die auf dem Prinzip der verdrillten Zelle, dem Guest-Host-Effekt, dem Effekt der Deformation aufgerichteter Phasen, dem Effekt der dynamischen Streuung oder dem 2-Frequenzverfahren beruhen.

Der Erfindung lag die Aufgabe zugrunde, neue stabile flüssigkristalline oder mesogene Verbindungen aufzufinden, die als Komponenten flüssigkristalliner Dielektrika geeignet sind. Diese Aufgabe wurde durch die Bereitstellung der Verbindung der Formel I gelöst.

Es wurde gefunden, dass die Verbindungen der Formel I als Komponenten flüssigkristalliner Dielektrika vorzüglich geeignet sind. Insbesondere sind mit ihrer Hilfe stabile flüssigkristalline Phasen mit stark negativer wie positiver dielektrischer Anisotropie und damit kleiner Schwellen- bzw. Steuerspannung elektrooptischer Effekte, sehr variabler optischer Anisotropie und vergleichsweise niedriger Viskosität herstellbar.

Mit der Bereitstellung der Verbindungen der Formel I wird ausserdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums wesentlich zu beeinflussen. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristallline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, dass man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,.

oder dass man an eine Verbindung der Formel II

$$R^1-(A^1)_m-Z^1-CH(CGOH)-CH_2CH_2-CHOH-Z^2-(A^2)_n-R^2 \quad II$$

worin

G, $R^1$, $R^2$, $A^1$, $A^2$, $Z^1$, $Z^2$, m und n die angegebenen Bedeutungen haben

oder eines ihrer reaktionsfähigen Derivate cyclisiert,

oder dass man eine Verbindung der Formel III

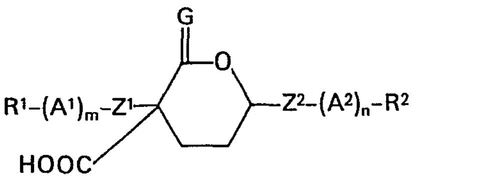

III

worin

G, $R^1$, $R^2$, $A^1$, $A^2$, $Z^1$, $Z^2$, m und n die angegebenen Bedeutungen haben decarboxyliert,

oder dass man zur Herstellung von Verbindungen der Formel I, worin $R^1$ und/oder $R^2$ F, Cl, Br oder CN bedeuten, in einem entsprechenden Diazoniumsalz die Diazoniumgruppe durch F, Cl, Br oder CN ersetzt,

oder dass man zur Herstellung von Estern der Formel I (worin $Z^1$ und/oder $Z^2$ -CO-O oder -O-CO- bedeuten) eine entsprechende Carbonsäure oder eines ihrer reaktionsfähigen Derivate mit einem entsprechenden Alkohol oder einem seiner reaktionsfähigen Derivate umsetzt,

oder dass man zur Herstellung von Dioxanderivaten der Formel I (worin $A^1$ und/oder $A^2$ 1,3-Dioxan-2,5-diyl bedeuten) einen entsprechenden Aldehyd mit einem entsprechenden Diol umsetzt,

oder dass man zur Herstellung von Nitrilen der Formel I (worin $R^1$ und/oder $R^2$ CN bedeuten) ein entsprechendes Carbonsäureamid dehydratisiert oder ein entsprechendes Carbonsäurehalogenid mit Sulfamid umsetzt,

oder dass man zur Herstellung von Ethern der Formel I (worin $R^1$ und/oder $R^2$ Alkylketten bedeuten, worin eine oder zwei $CH_2$-Gruppen durch O-Atome ersetzt sind, und/oder $Z^1$ und/oder $Z^2$ $-OCH_2-$ oder $-CH_2O-$ Gruppen sind) eine entsprechende Hydroxyverbindung verethert,

und/oder dass man gegebenenfalls eine Chlor- oder Bromverbindung der Formel I (worin $R^1$ und/oder $R^2$ Cl oder Br bedeuten) mit einem Cyanid umsetzt,

und/oder dass man gegebenenfalls eine Base der Formel I durch Behandeln mit einer Säure in eines ihrer Säureadditionssalze umwandelt,

oder dass man gegebenenfalls eine Verbindung der Formel I aus einem ihrer Säureadditionssalze durch Behandeln mit einer Base freisetzt.

Weiterhin ist Gegenstand der Erfindung die Verwendung der Verbindungen der Formel I als Komponenten flüssigkristalliner Dielektrika. Gegenstand der Erfindung sind ferner flüssigkristalline Dielektrika mit einem Gehalt an mindestens einer Verbindung der Formel I sowie elektrooptische Anzeigeelemente, die derartige Dielektrika enthalten.

Vor- und nachstehend haben G, $R^1$, $R^2$, $A^1$, $A^2$, $Z^1$, $Z^2$, m und n die angegebene Bedeutung, sofern nicht ausdrücklich etwas anderes vermerkt ist.

Die Verbindungen der Formel I umfassen dementsprechend Verbindungen der Teilformeln Ia und Ib (mit jeweils zwei Ringen), Ic bis Ie (mit jeweils drei Ringen) sowie If bis Ii (mit jeweils vier Ringen):

| | |
|---|---|
| $R^1$-$A^1$-$Z^1$-A-$Z^2$-$R^2$ | Ia |
| $R^1$-$Z^1$-A-$Z^2$-$A^2$-$R^2$ | Ib |
| $R^1$-$(A^1)_2$-$Z^1$-A-$Z^2$-$R^2$ | Ic |
| $R^1$-$A^1$-$Z^1$-A-$Z^2$-$A^2$-$R^2$ | Id |
| $R^1$-$Z^1$-A-$Z^2$-$(A^2)_2$-$R^2$ | Ie |
| $R^1$-$(A^1)_3$-$Z^1$-A-$Z^2$-$R^2$ | If |
| $R^1$-$(A^1)_2$-$Z^1$-A-$Z^2$-$A^2$-$R^2$ | Ig |
| $R^1$-$A^1$-$Z^1$-A-$Z^2$-$(A^2)_2$-$R^2$ | Ih |
| $R^1$-$Z^1$-A-$Z^2$-$(A^2)_3$-$R^2$ | Ii. |

Die bevorzugten Verbindungen der Teilformeln Ia und Ib umfassen solche der Teilformeln Iaa bis Iaf sowie Iba bis Ibf

| | |
|---|---|
| $R^1$-Phe-$Z^1$-A-$Z^2$-$R^2$ | Iaa |
| $R^1$-Cy-$Z^1$-A-$Z^2$-$R^2$ | Iab |
| $R^1$-Dio-$Z^1$-A-$Z^2$-$R^2$ | Iac |
| $R^1$-Pip-$Z^1$-A-$Z^2$-$R^2$ | Iad |
| $R^1$-Bi-$Z^1$-A-$Z^2$-$R^2$ | Iae |
| $R^1$-Pyr-$Z^1$-A-$Z^2$-$R^2$ | Iaf |
| $R^1$-$Z^1$-A-$Z^2$-Phe-$R^2$ | Iba |
| $R^1$-$Z^1$-A-$Z^2$-Cy-$R^2$ | Ibb |
| $R^1$-$Z^1$-A-$Z^2$-Dio-$R^2$ | Ibc |
| $R^1$-$Z^1$-A-$Z^2$-Pip-$R^2$ | Ibd |
| $R^1$-$Z^1$-A-$Z^2$-Bi-$R^2$ | Ibe |
| $R^1$-$Z^1$-A-$Z^2$-Pyr-$R^2$ | Ibf |

Darunter sind diejenigen der Formel Iba besonders bevorzugt.

Von den Verbindungen der Teilformeln Ic bis Ii sind diejenigen der Teilformeln Ie und Ii besonders bevorzugt, im einzelnen diejenigen der Teilformeln Ica bis Iih:

| | |
|---|---|
| $R^1$-Phe-Phe-$Z^1$-A-$Z^2$-$R^2$ | Ica |
| $R^1$-Phe-Cy-$Z^1$-A-$Z^2$-$R^2$ | Icb |
| $R^1$-Cy-Phe-$Z^1$-A-$Z^2$-$R^2$ | Icc |
| $R^1$-Cy-Cy-$Z^1$-A-$Z^2$-$R^2$ | Icd |
| $R^1$-Phe-$Z^1$-A-$Z^2$-Phe-$R^2$ | Ida |
| $R^1$-Phe-$Z^1$-A-$Z^2$-Cy-$R^2$ | Idb |
| $R^1$-Cy-$Z^1$-A-$Z^2$-Phe-$R^2$ | Idc |
| $R^1$-Cy-$Z^1$-A-Z-Cy-$R^2$ | Idd |
| $R^1$-$Z^1$-A-$Z^2$-Phe-Phe-$R^2$ | Iea |
| $R^1$-$Z^1$-A-$Z^2$-Phe-Cy-$R^2$ | Ieb |
| $R^1$-$Z^1$-A-$Z^2$-Cy-Phe-$R^2$ | Iec |
| $R^1$-$Z^1$-A-$Z^2$-Cy-Cy-$R^2$ | Ied |
| $R^1$-Phe-Phe-Phe-$Z^1$-A-$Z^2$-$R^2$ | Ifa |
| $R^1$-Phe-Phe-Cy-$Z^1$-A-$Z^2$-$R^2$ | Ifb |
| $R^1$-Phe-Cy-Phe-$Z^1$-A-$Z^2$-$R^2$ | Ifc |
| $R^1$-Phe-Cy-Cy-$Z^1$-A-$Z^2$-$R^2$ | Ifd |
| $R^1$-Cy-Phe-Phe-$Z^1$-A-$Z^2$-$R^2$ | Ife |
| $R^1$-Cy-Phe-Cy-$Z^1$-A-$Z^2$-$R^2$ | Iff |
| $R^1$-Cy-Cy-Phe-$Z^1$-A-$Z^2$-$R^2$ | Ifg |
| $R^1$-Cy-Cy-Cy-$Z^1$-A-$Z^2$-$R^2$ | Ifh |
| $R^1$-Phe-Phe-$Z^1$-A-$Z^2$-Phe-$R^2$ | Iga |
| $R^1$-Phe-Phe-$Z^1$-A-$Z^2$-Cy-$R^2$ | Igb |
| $R^1$-Phe-Cy-$Z^1$-A-$Z^2$-Phe-$R^2$ | Igc |

$R^1$-Phe-Cy-$Z^1$-A-$Z^2$-Cy-$R^2$ Igd
$R^1$-Cy-Phe-$Z^1$-A-$R^2$-Phe-$R^2$ Ige
$R^1$-Cy-Phe-$Z^1$-A-$Z^2$-Cy-$R^2$ Igf
$R^1$-Cy-Cy-$Z^1$-A-$Z^2$-Phe-$R^2$ Igg
$R^1$-Cy-Cy-$Z^1$-A-$Z^2$-Cy-$R^2$ Igh

$R^1$-Phe-$Z^1$-A-$Z^2$-Phe-Phe-$R^2$ Iha
$R^1$-Phe-$Z^1$-A-$Z^2$-Phe-Cy-$R^2$ Ihb
$R^1$-Phe-$Z^1$-A-$Z^2$-Cy-Phe-$R^2$ Ihc
$R^1$-Phe-$Z^1$-A-$Z^2$-Cy-Cy-$R^2$ Ihd
$R^1$-Cy-$Z^1$-A-$Z^2$-Phe-Phe-$R^2$ Ihe
$R^1$-Cy-$Z^1$-A-$Z^2$-Phe-Cy-$R^2$ Ihf
$R^1$-Cy-$Z^1$-A-$Z^2$-Cy-Phe-$R^2$ Ihg
$R^1$-Cy-$Z^1$-A-$Z^2$-Cy-Cy-$R^2$ Ihh

$R^1$-$Z^1$-A-$Z^2$-Phe-Phe-Phe-$R^2$ Iia
$R^1$-$Z^1$-A-$Z^2$-Phe-Phe-Cy-$R^2$ Iib
$R^1$-$Z^1$-A-$Z^2$-Phe-Cy-Phe-$R^2$ Iic
$R^1$-$Z^1$-A-$Z^2$-Phe-Cy-Cy-$R^2$ Iid
$R^1$-$Z^1$-A-$Z^2$-Cy-Phe-Phe-$R^2$ Iie
$R^1$-$Z^1$-A-$Z^2$-Cy-Phe-Cy-$R^2$ Iif
$R^1$-$Z^1$-A-$Z^2$-Cy-Cy-Phe-$R^2$ Iig
$R^1$-$Z^1$-A-$Z^2$-Cy-Cy-Cy-$R^2$ Iih.

Besonders bevorzugt sind die Verbindungen der Teilformeln Iea und Iib.

In den Verbindungen der vor- und nachstehenden Formeln bedeuten $R^1$ und $R^2$ vorzugsweise Alkyl, ferner Alkoxy (insbesondere, wenn diese Reste an einer Phe-Gruppe stehen) oder eine andere Oxaalkylgruppe.

$A^1$ und $A^2$ sind bevorzugt Cy oder Phe, ferner bevorzugt Dio oder Pip; bevorzugt enthält die Verbindung der Formel I nicht mehr als einen der Reste Dio, Pip, Bi oder Pyr.

G steht bevorzugt für 2 H-Atome.

$Z^1$ und $Z^2$ sind bevorzugt Einfachbindungen, in zweiter Linie bevorzugt -CO-O oder-O-CO-Gruppen.

m ist vorzugsweise O, n ist vorzugsweise I.

In den Verbindungen der vor- und nachstehenden Formeln können die Alkylreste, in denen auch eine («Alkoxy» bzw. «Oxaalkyl») oder zwei («Alkoxyalkoxy» bzw. «Dioxaalkyl») $CH_2$-Gruppen durch O-Atome ersetzt sein können, geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (=Methoxymethyl), 2- (=Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formeln I sowie Ia bis Iih mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Unter den Verbindungen der Formeln I sowie Ia bis Iih sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln Ij bis Iq

$R^1$-A-$Z^2$-$A^2$-$R^2$. Ij
$R^1$-A-$A^2$-$R^2$ Ik
$R^1$-A-Phe-$R^2$ Il
$R^1$-A-Phe-CN Im
$R^1$-A-$(A^2)_2$-$R^2$ In
$R^1$-A-Phe-Phe-$R^2$ Io
$R^1$-A-$(A^2)_3$-$R^2$ Ip
$R^1$-A-Phe-Phe-Cy-$R^2$ Iq

Diejenigen der vorstehend genannten Formeln, die eine oder mehrere der Gruppen Dio, Pip und/oder Pyr enthalten, umschliessen jeweils die beiden möglichen 2,5- bzw. 1,4-Stellungsisomeren. So umschliesst beispielsweise die Teilformel Iac die 2-$R^1$-5-(A-$R^2$)-1,3-dioxane und die 2-(A-$R^2$)-5-$R^1$-1,3-dioxane, die Teilformel Iad die 1-$R^1$-4-(A-$R^2$)-piperidine und die 1-(A-$R^2$)-4-$R^1$-piperidine.

Alle genannten Verbindungen, die Cyclohexan-1,3-Dioxan-und/oder Tetrahydropyranringe enthalten, können als cis- und als trans-Formen sowie als Gemische vorliegen.

Diejenigen Verbindungen, in denen die Substituenten in trans-Stellung zueinander stehen, sind bevorzugt. Diese sind in der Regel stabiler; in vielen Fällen lassen sich die cis-Verbindungen (oder Gemische) durch Behandeln mit einer Base, z.B. mit K-tert.-Butylat in einem inerten Lösungsmittel wie Dimethylsulfoxid, in die trans-Verbindungen umwandeln.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, dass man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle des Tetrahydropyranrings einen Dihydropyranring und/oder an Stelle eines Cyclohexanrings einen Cyclohexenring oder Cyclohexanonring und/oder an Stelle einer $-CH_2CH_2-$ Gruppe eine $-CH=CH-$ Gruppe oder eine $-CH_2-CO-$ Gruppe enthalten.

Die Reduktion erfolgt zweckmässig durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan.

Als Katalysatoren eignen sich zweckmässig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form (z.B. Pt-Mohr) eingesetzt werden können.

Besonders vorteilhaft werden die Verbindungen der Formel I durch Cyclisierung der Verbindungen der Formel II oder ihrer reaktionsfähigen Derivate erhalten. Lactonisierung der Hydroxysäuren der Formel II (G=O) führt zu den Lactonen der Formel I (G=O); dehydratisierende Cyclisierung der Diole der Formel II ($G=H_2$) führt zu den Tetrahydropyranen der Formel I ($G=H_2$).

Die Ausgangsstoffe der Formel II sind beispielsweise erhältlich durch Reaktionen von Malonsäureestern der Formel

$R^1-(A^1)_m-Z^1-CH(COOAlkyl)_2$ mit Halogeniden der Formel $Hal-CH_2CH_2-CO-Z^2-(A^2)_n-R^2$ (Hal=Cl, Br) zu Verbindungen der Formel

$R^1-(A^1)_m-Z^1-C(COOAlkyl)_2-CH_2CH_2-CO-Z^2-(A^2)_n-R^2$,

Verseifung und Decarboxylierung zu Ketosäuren der Formel

$R^1-(A^1)_m-Z^1-CH(COOH)-CH_2CH_2-CO-Z^2-(A^2)_n-R^2$

sowie Reduktion zu den Hydroxysäuren II (G=O), die meist nicht isoliert werden, sondern spontan lactonisieren, oder zu den Diolen II ($G=H_2$).

Als reaktionsfähige Derivate der Verbindungen der Formel II eignen sich z.B. die entsprechenden Halogenderivate (Hal an Stelle einer oder beider OH-Gruppen), ferner entsprechende reaktive Ester, z.B. Alkyl- oder Arylsulfonate, worin die Alkylgruppen insbesondere 1-6, die Arylgruppen 6-10 C-Atome enthalten, z.B. die Mono- oder Dimethan-, -benzol- oder -p-toluolsulfonate der genannten Diole.

Die Cyclisierung erfolgt in der Regel bei Temperaturen zwischen etwa 0 und 250° in Gegenwart oder Abwesenheit eines inerten Lösungsmittels, z.B. eines Kohlenwasserstoffs wie Benzol, Toluol oder Xylol, eines Amids wie Dimethylformamid oder Phosphorsäure-hexamethyl-triamid oder eines Sulfoxids wie Dimethylsulfoxid. Die Lactonisierung gelingt in der Regel durch einfaches Erhitzen auf etwa 150-250°. Die Cyclisierung der Diole erfolgt zweckmässig bei etwa 60-150° in Gegenwart eines sauren Katalysators, z.B. einer Mineralsäure wie Schwefelsäure, Salzsäure, Phosphorsäure, Perchlorsäure, einer organischen Sulfonsäure wie Benzol-, p-Toluol- oder Naphthalinsulfonsäure, einer Lewissäure wie $BF_3$ oder $ZnCl_2$ oder eines sauren Salzes wie $NaHSO_4$.

Die Verbindungen der Formel I können ferner durch Decarboxylierung der Verbindungen der Formel III erhalten werden. In diesen ist G bevorzugt ein O-Atom. Sie sind beispielsweise erhältlich durch Reduktion der genannten Keto-malonester der Formel

$R^1-(A^1)_m-Z^1-C(COOAlkyl)_2-CH_2CH_2-CO-Z^2-(A^2)_n-R^2$

zu den entsprechenden Hydroxyestern der Formel

$R^1-(A^1)_m-Z^1-C(COOAlkyl)_2-CH_2CH_2-CHOH-Z^2-(A^2)_n-R^2$,

die bei der Verseifung sehr leicht zu den Valerolactoncarbonsäuren der Formel III (G=O) lactonisieren.

In der Regel werden die Verbindungen der Formel III nicht isoliert, sondern durch Erhitzen in Abwesenheit oder in Gegenwart eines inerten Lösungsmittels auf Temperaturen zwischen etwa 80 und 250° decarboxyliert.

Verbindungen der Formel I, worin $R^1$ und/oder $R^2$ F, Cl, Br oder CN bedeuten, können auch aus den entsprechenden Diazoniumsalzen durch Austausch der Diazoniumgruppe gegen ein Fluor-, Chlor- oder Bromatom oder gegen eine CN-Gruppe, z.B. nach den Methoden von Schiemann oder Sandmeyer, erhalten werden.

Die Diazoniumsalze sind z.B herstellbar durch Nitrierung von Verbindungen, die der Formel I entsprechen, aber an Stelle der Reste $R^1$ und/oder $R^2$ ein (oder zwei) Wasserstoffatom(e) enthalten, Reduktion zu den entsprechenden Aminen und Diazotierung beispielsweise mit $NaNO_2$ oder $KNO_2$ in wässriger Lösung bei Temperaturen zwischen etwa $-10$ und $+10°$.

Zum Austausch der Diazoniumgruppe gegen Fluor kann man in wasserfreier Flussäure diazotieren und anschliessend erwärmen, oder man setzt mit Tetrafluorborsäure zu den Diazoniumtetrafluorboraten um, die anschliessend thermisch zersetzt werden.

Ein Austausch gegen Cl, Br oder CN gelingt zweckmässig durch Reaktion der wässrigen Diazoniumsalzlösung mit $Cu_2Cl_2$, $Cu_2Br_2$ oder $Cu_2(CN)_2$ nach der Methode von Sandmeyer.

Ester der Formel I ($Z^1$ und/oder $Z^2$ = $-CO-O-$ oder $-O-CO-$) können auch durch Veresterung entsprechender Carbonsäuren der Formeln

$R^1-(A^1)_m-COOH$, $R^1-(A^1)_m-Z^1-A-COOH$,

$R^2-(A^2)_n-COOH$ oder

reaktionsfähigen Derivate) mit Alkoholen bzw. Phenolen der Formeln $R^2-(A^2)_n-Z^2-A-OH$, $R^2-(A^2)_n-OH$, $R^1-(A^1)_m-Z^1-A-OH$ oder $R^1-(A^1)_m-OH$ (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride der Formeln $R^1-(A^1)_m-CO-O-COCH_3$, $R^1-(A^1)_m-Z^1-A-CO-O-COCH_3$, $R^2-(A^2)_n-CO-O-COCH_3$ und $R^2-(A^2)_n-Z^2-A-CO-O-COCH_3$, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate der Formeln $R^2-(A^2)_n-Z^2-A-OM$, $R^2-(A^2)_n-OM$, $R^1-(A^1)_m-Z^1-A-OM$ und $R^1-(A^1)_m-OM$ in Betracht, worin M ein Äquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuss einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen −50° und +250°, vorzugsweise zwischen −20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, dass man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmässig bei Temperaturen zwischen etwa −25° und +20°.

Dioxanderivate der Formel I (worin eine der Gruppen $A^1$ und/oder $A^2$ eine 1,3-Dioxan-2,5-diyl-Gruppe bedeutet) werden zweckmässig durch Reaktion eines entsprechenden Aldehyds, z.B. der Formeln $R^1-(A^1)_{m-1}-CHO$, $R^1-(A^1)_m-Z^1-A-Z^2-CHO$, $O=CH-(A^1)_{m-1}-Z^1-A-Z^2-(A^2)_n-R^2$ bzw. $O=CH-R^2$ (oder eines seiner reaktionsfähigen Derivate) mit einem entsprechenden 1,3-Diol z.B. der Formeln

$(HOCH_2)_2CH-(A^1)_{m-1}-Z^1-A-Z^2-(A^2)_n-R^2$,
$(HOCH_2)_2CH-R^2$, $R^1-(A^1)_{m-1}-CH(CH_2OH)_2$ bzw.
$R^1-(A^1)_m-Z^1-A-Z^2-CH(CH_2OH)_2$

(oder einem seiner reaktionsfähigen Derivate) hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale z.B. der Formeln

$R^1-(A^1)_{m-1}CH(OR^3)_2$,
$R^1-(A^1)_m-Z^1-A-Z^2-CH(OR^3)_2$,
$(R^3O)_2CH-(A^1)_{m-1}-Z^1-A-Z^2-(A^2)_n-R^2$,
$(R^3O)_2-R^2$,
$R_4-CH(OCH_2)_2CH-(A^1)_{m-1}-Z^1-A-Z^2-(A^2)_n-R^2$,
$R_4-CH(OCH_2)_2CH-R^2$,
$R^1-(A^1)_{m-1}-CH(CH_2O)_2CH-R^4$ bzw.
$R^1-(A^1)_m-Z^1-A-Z^1-CH(CH_2O)_2CHR^4$,

worin $R^3$ Alkyl mit 1-4 C-Atomen, zwei Reste $R^3$ zusammen auch Alkylen mit 2 oder 3 C-Atomen und $R^4$ H, Alkyl mit 1-4 C-Atomen oder Phenyl bedeuten.

Die genannten Aldehyde und 1,3-Diole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester erhältlich.

Zur Herstellung von Nitrilen der Formel I (worin $R^1$ und/oder $R^2$ CN bedeuten) können entspre-

chende Säureamide, z.B. solche, in denen an Stelle des Restes $R^1$ und/oder $R^2$ eine $CONH_2$-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$ $COCl_2$, ferner $P_2O_5$, $P_2S_5$, $AlCl_3$ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmässig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I (worin $R^1$ und/oder $R^2$ eine Alkylkette bedeuten, worin eine oder zwei $CH_2$-Gruppen durch O-Atome ersetzt sind, und/ oder worin $Z^1$ und/oder $Z^2$ eine $-OCH_2-$ oder eine $-CH_2O-$ Gruppe ist) sind durch Veretherung entsprechender Hydroxyverbindungen, vorzugsweise entsprechender Phenole, erhältlich, wobei die Hydroxyverbindung zweckmässig zunächst in ein entsprechendes Metallderivat, z.B. durch Behandeln mit NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmässig in einem inerten Lösungsmittel wie Aceton, DMF oder Dimethylsulfoxid oder auch einem Überschuss an wässriger oder wässrig-alkoholischer NaOH oder KOH bei Temperaturen zwischen etwa 20° und 100°.

Zur Herstellung von Nitrilen der Formel I (worin $R^1$ und/oder $R^2$ CN bedeuten) können auch entsprechende Chlor- oder Bromverbindungen der Formel I (worin $R^1$ und/oder $R^2$ Cl oder Br bedeuten) mit einem Cyanid umgesetzt werden, zweckmässig mit einem Metallcyanid wie NaCN, KCN oder $Cu_2(CN)_2$, z.B. in Gegenwart von Pyridin in einem inerten Lösungsmittel wie DMF oder N-Methylpyrrolidon bei Temperaturen zwischen 20° und 200°.

Eine Base der Formel I kann mit einer Säure in das zugehörige Säureadditionssalz übergeführt werden. Für diese Umsetzung können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere alipathische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, naphthalinmono- und disulfonsäuren, Laurylschwefelsäure.

Umgekehrt ist es möglich, aus einem Säureadditionssalz einer Verbindung der Formel I die Base der Formel I durch Behandeln mit einer Base freizusetzen, z.B. mit einer starken anorganischen Base wie KOH oder NaOH.

Die erfindungsgemässen Dielektrika bestehen aus 2 bis 20, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, gegebenenfalls halogenierten Stilbene, Benzylphenylether, Benzyl-cyclohexylether, Cyclohexylmethyl-phenylether, Cyclohexylmethyl-cyclohexylether, Tolane, substituierten Zimtsäuren, Dekaline, Perhydrophenanthrene, Bi-cyclooctane, 1,2-Di-cyclohexylethane, 1-Cyclohexyl-2-phenylethane.

Die wichtigsten als Bestandteile derartiger flüssigkristalliner Dielektrika in Frage kommenden Verbindungen lassen sich durch die Formel IV charakterisieren,

$$R'-X-Y-Z-R'' \qquad IV$$

worin X und Z je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| Y | $-CH=CH-$ | $-N(O)=N-$ |
| | $-CH=CQ-$ | $-CH=N(O)-$ |
| | $-C\equiv C-$ | $-CH_2-CH_2-$ |
| | $-CO-O-$ | $-CH_2-O-$ |
| | $-CO-S-$ | $-CH_2-S-$ |
| | $-CH=N-$ | $-COO-Phe-COO-$ |

oder eine C-C-Einfachbindung, Q Halogen, vorzugsweise Chlor, oder -CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R″ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemässen Dielektrika enthalten etwa 0,1 bis 100, vorzugsweise 10 bis 100%, einer oder mehrerer Verbindungen der Formel I.

Die Herstellung der erfindungsgemässen Dielektrika erfolgt in an sich üblicher Weise. In der Regel werden die Komponenten ineinander gelöst, zweckmässig bei erhöhter Temperatur.

Durch geeignete Zusätze können die flüssigkristallinen Dielektrika nach der Erfindung so modifiziert werden, dass sie in allen bisher bekannt gewordenen Arten von Flüssigkristallanzeigeelementen verwendet werden können.

Derartige Zusätze sind dem Fachmann geläufig und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst. Liq. Cryst. Band 24, Seiten 249–258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-A-2 209 127, 2 240 864, 2 321 632, 2 338 281, 2 450 088, 2 637 430, 2 853 728 und 2 902 177 beschrieben.

Die folgenden Beispiele sollen die Erfindung erläutern, ohne sie zu begrenzen. F. = Schmelzpunk, K. = Klärpunkt. Vor- und nachstehend bedeuten Prozentangaben Gewichtsprozent; alle Temperaturen sind in Grad Celsius angegeben. «Übliche Aufarbeitung» bedeutet: man gibt Wasser hinzu, extrahiert mit Methylenchlorid, trennt ab, trocknet die organische Phase, dampft ein und reinigt das Produkt durch Kristallisation und/oder Chromatographie.

Beispiel 1

Eine Lösung von 23,4 g 3-(p-Methoxybenzoylmethyl)-tetrahydropyran (erhältlich durch Reaktion von 2,3-Dihydro-4H-pyran mit HBr zu 3-Bromtetrahydropyran, Reaktion mit Malonsäurediethylester zu Tetrahydropyran-3-ylmalonsäure-diethylester, Verseifung, Decarboxylierung, Umsetzung mit $SOCl_2$ zu Tetrahydropyran-3-acetylchlorid und Reaktion mit Anisol in Gegenwart von $AlCl_3$) in 500 ml THF wird an 5 g 10%igem PdC bei 40° und 1 bar bis zur Aufnahme von 0,2 Mol $H_2$ hydriert. Man filtriert, dampft ein und erhält 3-(2-p-Methoxyphenylethyl)-tetrahydropyran.

Analog sind erhältlich:
3-(2-p-Ethoxyphenylethyl)-tetrahydropyran.
3-(2-p-Tolylethyl)-tetrahydropyran.
3-(2-p-Ethylphenylethyl)-tetrahydropyran.
3-(2-p-Propylphenylethyl)-tetrahydropyran.
3-(2-p-Butylphenylethyl)-tetrahydropyran.
3-(2-p-Pentylphenylethyl)-tetrahydropyran.
3-(2-p-Hexylphenylethyl)-tetrahydropyran.

Beispiel 2

Man löst 10 g 3-p-Propylphenyl-4,5-dihydro-6H-pyran (erhältlich durch Hydrolyse von 3-Bromtetrahydropyran zu 3-Hydroxytetrahydropyran, Reaktion mit p-Propylphenylmagnesiumbromid zu 3-p-Propylphenyl-3-hydroxy-tetrahydropyran und anschliessend Dehydratisierung) in 100 ml Ethanol und hydriert bei 3 at und 20° an 2 g Raney-Nickel bis zum Stillstand. Nach Dekantieren und Eindampfen erhält man 3-p-Propylphenyl-tetrahydropyran.

Analog sind erhältlich:
3-p-Tolyl-tetrahydropyran.
3-p-Ethylphenyl-tetrahydropyran.
3-p-Butylphenyl-tetrahydropyran.
3-p-Pentylphenyl-tetrahydropyran.
3-p-Methoxyphenyl-tetrahydropyran.
3-p-Ethoxyphenyl-tetrahydropyran.

Beispiel 3

Man kocht eine Lösung von 29,4 g 4-Hydroxymethyl-1-p-ethoxyphenyl-nonan-1-ol [erhältlich durch Reaktion von 2-Pentylmalonsäure-diethylester (KP. 127°/13mbar) mit 3-Brompropionsäureethylester zu 2-Carbethoxy-2-pentylglutarsäurediethylester (Kp. 160°/0,4 mbar), Verseifung und Decarboxylierung zu 2-Pentylglutarsäure, Erhitzen unter Bildung des Anhydrids (Kp 120°/0,7 mbar), Reaktion mit Phenetol/$AlCl_3$ zu 4-p-Ethoxybenzoyl-2-pentyl-buttersäure (F. 61°) und Reduktion mit $LiAlH_4$] und 1 g p-Toluolsulfonsäure in 400 ml Benzol 6 Stunden mit Wasserabscheider, wäscht mit $NaHCO_3$-Lösung, trocknet, dampft ein und erhält 2-p-Ethoxyphenyl-5-pentyl-tetrahydropyran.

Analog sind erhältlich:
2-m-Fluorphenyl-5-pentyl-tetrahydropyran
2-(2,3-Difluorphenyl)-5-pentyl-tetrahydropyran
2-p-Methoxyphenyl-5-propyl-tetrahydropyran
2-p-Methoxyphenyl-5-butyl-tetrahydropyran
2-p-Methoxyphenyl-5-pentyl-tetrahydropyran
2-p-Methoxyphenyl-5-hexyl-tetrahydropyran
2-p-Methoxyphenyl-5-heptyl-tetrahydropyran
2-p-Methoxyphenyl-5-octyl-tetrahydropyran
2-p-Ethoxyphenyl-5-propyl-tetrahydropyran
2-p-Ethoxyphenyl-5-butyl-tetrahydropyran
2-p-Ethoxyphenyl-5-hexyl-tetrahydropyran
2-p-Ethoxyphenyl-5-heptyl-tetrahydropyran
2-p-Ethoxyphenyl-5-octyl-tetrahydropyran
2-p-Propoxyphenyl-5-propyl-tetrahydropyran
2-p-Propoxyphenyl-5-butyl-tetrahydropyran
2-p-Propoxyphenyl-5-pentyl-tetrahydropyran
2-p-Propoxyphenyl-5-hexyl-tetrahydropyran
2-p-Propoxyphenyl-5-heptyl-tetrahydropyran
2-p-Propoxyphenyl-5-octyl-tetrahydropyran

Beispiel 4

Analog Beispiel 3 erhält man aus 2-Phenyl-octan-1,5-diol [erhältlich durch Reaktion von Butyronitril mit 2-Tetrahydropyranyloxy-ethylmagnesiumbromid zu 1-Tetrahydropyranyloxy-hexan-3-on, Etherspaltung, Ketalisierung und Umsetzung mit $PBr_3$ zu 1-Bromhexan-3-on-ethylenketal, Reaktion mit Phenylmalonsäurediethylester zu Phenyl-(3-oxohexyl)-malonsäurediethylester, Verseifung und Decarboxylierung zu 2-Phenyl-5-oxo-octansäure und Reduktion mit $LiAlH_4$] das 2-Propyl-5-phenyl-tetrahydropyran

Analog sind erhältlich:
2-Butyl-5-phenyl-tetrahydropyran
2-Pentyl-5-phenyl-tetrahydropyran
2-Hexyl-5-phenyl-tetrahydropyran
2-Heptyl-5-phenyl-tetrahydropyran
2-Octyl-5-phenyl-tetrahydropyran
2-Propyl-5-p-methoxyphenyl-tetrahydropyran
2-Butyl-5-p-methoxyphenyl-tetrahydropyran
2-Pentyl-5-p-methoxyphenyl-tetrahydropyran
2-Hexyl-5-p-methoxyphenyl-tetrahydropyran
2-Heptyl-5-p-methoxyphenyl-tetrahydropyran
2-Octyl-5-p-methoxyphenyl-tetrahydropyran
2-Propyl-5-p-ethoxyphenyl-tetrahydropyran
2-Butyl-5-p-ethoxyphenyl-tetrahydropyran
2-Pentyl-5-p-ethoxyphenyl-tetrahydropyran
2-Hexyl-5-p-ethoxyphenyl-tetrahydropyran
2-Heptyl-5-p-ethoxyphenyl-tetrahydropyran
2-Octyl-5-p-ethoxyphenyl-tetrahydropyran
2-Propyl-5-p-tolyl-tetrahydropyran
2-Butyl-5-p-tolyl-tetrahydropyran
2-Pentyl-5-p-tolyl-tetrahydropyran
2-Hexyl-5-p-tolyl-tetrahydropyran
2-Heptyl-5-p-tolyl-tetrahydropyran
2-Octyl-5-p-tolyl-tetrahydropyran
2-Propyl-5-p-ethylphenyl-tetrahydropyran
2-Butyl-5-p-ethylphenyl-tetrahydropyran
2-Pentyl-5-p-ethylphenyl-tetrahydropyran
2-Hexyl-5-p-ethylphenyl-tetrahydropyran
2-Heptyl-5-p-ethylphenyl-tetrahydropyran
2-Octyl-5-p-ethylphenyl-tetrahydropyran
2-Propyl-5-p-propylphenyl-tetrahydropyran
2-Butyl-5-p-propylphenyl-tetrahydropyran
2-Pentyl-5-p-propylphenyl-tetrahydropyran
2-Hexyl-5-p-propylphenyl-tetrahydropyran
2-Heptyl-5-p-propylphenyl-tetrahydropyran
2-Octyl-5-p-propylphenyl-tetrahydropyran
2-Propyl-5-p-butylphenyl-tetrahydropyran
2-Butyl-5-p-butylphenyl-tetrahydropyran
2-Pentyl-5-p-butylphenyl-tetrahydropyran
2-Hexyl-5-p-butylphenyl-tetrahydropyran
2-Heptyl-5-p-butylphenyl-tetrahydropyran
2-Octyl-5-p-butylphenyl-tetrahydropyran
2-Propyl-5-p-pentylphenyl-tetrahydropyran
2-Butyl-5-p-pentylphenyl-tetrahydropyran
2-Pentyl-5-p-pentylphenyl-tetrahydropyran
2-Hexyl-5-p-pentylphenyl-tetrahydropyran
2-Heptyl-5-p-pentylphenyl-tetrahydropyran
2-Octyl-5-p-pentylphenyl-tetrahydropyran
2-Propyl-5-p-hexylphenyl-tetrahydropyran
2-Butyl-5-p-hexylphenyl-tetrahydropyran
2-Pentyl-5-p-hexylphenyl-tetrahydropyran
2-Hexyl-5-p-hexylphenyl-tetrahydropyran
2-Heptyl-5-p-hexylphenyl-tetrahydropyran
2-Octyl-5-p-hexylphenyl-tetrahydropyran
2-Propyl-5-p-heptylphenyl-tetrahydropyran
2-Butyl-5-p-heptylphenyl-tetrahydropyran
2-Pentyl-5-p-heptylphenyl-tetrahydropyran
2-Hexyl-5-p-heptylphenyl-tetrahydropyran
2-Heptyl-5-p-heptylphenyl-tetrahydropyran
2-Octyl-5-p-heptylphenyl-tetrahydropyran
2-Propyl-5-p-octylphenyl-tetrahydropyran
2-Butyl-5-p-octylphenyl-tetrahydropyran
2-Pentyl-5-p-octylphenyl-tetrahydropyran
2-Hexyl-5-p-octylphenyl-tetrahydropyran
2-Heptyl-5-p-octylphenyl-tetrahydropyran
2-Octyl-5-p-octylphenyl-tetrahydropyran
2-Propyl-5-p-fluorphenyl-tetrahydropyran
2-Butyl-5-p-fluorphenyl-tetrahydropyran
2-Pentyl-5-p-fluorphenyl-tetrahydropyran
2-Hexyl-5-p-fluorphenyl-tetrahydropyran
2-Heptyl-5-p-fluorphenyl-tetrahydropyran
2-Octyl-5-p-fluorphenyl-tetrahydropyran
2-Propyl-5-p-chlorphenyl-tetrahydropyran
2-Butyl-5-p-chlorphenyl-tetrahydropyran
2-Pentyl-5-p-chlorphenyl-tetrahydropyran
2-Hexyl-5-p-chlorphenyl-tetrahydropyran
2-Heptyl-5-p-chlorphenyl-tetrahydropyran
2-Octyl-5-p-chlorphenyl-tetrahydropyran
2-Propyl-5-p-bromphenyl-tetrahydropyran
2-Butyl-5-p-bromphenyl-tetrahydropyran
2-Pentyl-5-p-bromphenyl-tetrahydropyran
2-Hexyl-5-p-bromphenyl-tetrahydropyran
2-Heptyl-5-p-bromphenyl-tetrahydropyran
2-Octyl-5-p-bromphenyl-tetrahydropyran
2-Propyl-5-(4-biphenylyl)-tetrahydropyran
2-Butyl-5-(4-biphenylyl)-tetrahydropyran
2-Pentyl-5-(4-biphenylyl)-tetrahydropyran
2-Hexyl-5-(4-biphenylyl)-tetrahydropyran
2-Heptyl-5-(4-biphenylyl)-tetrahydropyran
2-Octyl-5-(4-biphenylyl)-tetrahydropyran

Beispiel 5

Man erhitzt 3,16 g rohe 2-Hexyl-2-(3-hydroxy-3-p-pentylphenyl-propyl)-malonsäure [ölig; erhältlich durch Friedel-Crafts-Reaktion von Pentylbenzol mit 3-Brompropionylchlorid zu p-Pentyl-ω-brompropiophenon, Reaktion mit 2-Hexylmalonsäurediethylester zu 2-Hexyl-2-(3-oxo-3-p-pentylphenyl-propyl)-malonsäurediethylester, $NaBH_4$-Reduktion zu 2-Hexyl-2-(3-hydroxy-3-p-pentylphenylpropyl)-malonsäurediethylester und Verseifung] 30 Minuten lang auf 210°. Die intermediär durch Cyclisierung bzw. Decarboxylierung entstehenden Verbindungen 2-Oxo-3-hexyl-6-p-pentylphenyl-tetrahydropyran-3-carbonsäure und 2-(3-Hydroxy-3-p-pentylphenyl-propyl)-octansäure werden nicht isoliert. Nach üblicher Aufarbeitung erhält man 2-Oxo-3-hexyl-6-p-pentylphenyl-tetrahydropyran, F. 101–102°.

Analog sind erhältlich:
2-Oxo-3-propyl-6-phenyl-tetrahydropyran
2-Oxo-3-butyl-6-phenyl-tetrahydropyran
2-Oxo-3-pentyl-6-phenyl-tetrahydropyran
2-Oxo-3-hexyl-6-phenyl-tetrahydropyran
2-Oxo-3-heptyl-6-phenyl-tetrahydropyran
2-Oxo-3-octyl-6-phenyl-tetrahydropyran

2-Oxo-3-propyl-6-p-tolyl-tetrahydropyran
2-Oxo-3-butyl-6-p-tolyl-tetrahydropyran
2-Oxo-3-pentyl-6-p-tolyl-tetrahydropyran
2-Oxo-3-hexyl-6-p-tolyl-tetrahydropyran
2-Oxo-3-heptyl-6-p-tolyl-tetrahydropyran
2-Oxo-3-octyl-6-p-tolyl-tetrahydropyran
2-Oxo-3-propyl-6-p-ethylphenyl-tetrahydropyran
2-Oxo-3-butyl-6-p-ethylphenyl-tetrahydropyran
2-Oxo-3-pentyl-6-p-ethylphenyl-tetrahydropyran
2-Oxo-3-hexyl-6-p-ethylphenyl-tetrahydropyran
2-Oxo-3-heptyl-6-p-ethylphenyl-tetrahydropyran
2-Oxo-3-octyl-6-p-ethylphenyl-tetrahydropyran
2-Oxo-3-propyl-6-p-propylphenyl-tetrahydropyran
2-Oxo-3-butyl-6-p-propylphenyl-tetrahydropyran
2-Oxo-3-pentyl-6-p-propylphenyl-tetrahydropyran
2-Oxo-3-hexyl-6-p-propylphenyl-tetrahydropyran
2-Oxo-3-heptyl-6-p-propylphenyl-tetrahydropyran
2-Oxo-3-octyl-6-p-propylphenyl-tetrahydropyran
2-Oxo-3-propyl-6-p-butylphenyl-tetrahydropyran
2-Oxo-3-butyl-6-p-butylphenyl-tetrahydropyran
2-Oxo-3-pentyl-6-p-butylphenyl-tetrahydropyran
2-Oxo-3-hexyl-6-p-butylphenyl-tetrahydropyran
2-Oxo-3-heptyl-6-p-butylphenyl-tetrahydropyran
2-Oxo-3-octyl-6-p-butylphenyl-tetrahydropyran
2-Oxo-3-propyl-6-p-pentylphenyl-tetrahydropyran
2-Oxo-3-butyl-6-p-pentylphenyl-tetrahydropyran
2-Oxo-3-pentyl-6-p-pentylphenyl-tetrahydropyran
2-Oxo-3-heptyl-6-p-pentylphenyl-tetrahydropyran
2-Oxo-3-octyl-6-p-pentylphenyl-tetrahydropyran
2-Oxo-3-propyl-6-p-hexylphenyl-tetrahydropyran
2-Oxo-3-butyl-6-p-hexylphenyl-tetrahydropyran
2-Oxo-3-pentyl-6-p-hexylphenyl-tetrahydropyran
2-Oxo-3-hexyl-6-p-hexylphenyl-tetrahydropyran
2-Oxo-3-heptyl-6-p-hexylphenyl-tetrahydropyran
2-Oxo-3-octyl-6-p-hexylphenyl-tetrahydropyran
2-Oxo-3-propyl-6-p-heptylphenyl-tetrahydropyran
2-Oxo-3-butyl-6-p-heptylphenyl-tetrahydropyran
2-Oxo-3-pentyl-6-p-heptylphenyl-tetrahydropyran
2-Oxo-3-hexyl-6-p-heptylphenyl-tetrahydropyran
2-Oxo-3-heptyl-6-p-heptylphenyl-tetrahydropyran
2-Oxo-3-octyl-6-p-heptylphenyl-tetrahydropyran
2-Oxo-3-propyl-6-p-octylphenyl-tetrahydropyran
2-Oxo-3-butyl-6-p-octylphenyl-tetrahydropyran
2-Oxo-3-pentyl-6-p-octylphenyl-tetrahydropyran
2-Oxo-3-hexyl-6-p-octylphenyl-tetrahydropyran
2-Oxo-3-heptyl-6-p-octylphenyl-tetrahydropyran
2-Oxo-3-octyl-6-p-octylphenyl-tetrahydropyran
2-Oxo-3-propyl-6-p-methoxyphenyl-tetrahydropyran
2-Oxo-3-butyl-6-p-methoxyphenyl-tetrahydropyran
2-Oxo-3-pentyl-6-p-methoxyphenyl-tetrahydropyran
2-Oxo-3-hexyl-6-p-methoxyphenyl-tetrahydropyran
2-Oxo-3-heptyl-6-p-methoxyphenyl-tetrahydropyran
2-Oxo-3-octyl-6-p-methoxyphenyl-tetrahydropyran
2-Oxo-3-propyl-6-p-ethoxyphenyl-tetrahydropyran
2-Oxo-3-butyl-6-p-ethoxyphenyl-tetrahydropyran
2-Oxo-3-pentyl-6-p-ethoxyphenyl-tetrahydropyran
2-Oxo-3-hexyl-6-p-ethoxyphenyl-tetrahydropyran
2-Oxo-3-heptyl-6-p-ethoxyphenyl-tetrahydropyran
2-Oxo-3-octyl-6-p-ethoxyphenyl-tetrahydropyran

Beispiel 6

Ein Gemisch von 2,34 g 2-Phenyl-5-oxo-octansäure, 0,3 g $NaBH_4$, 0,5 g NaOH und 25 ml Methanol wird 5 Stunden gekocht und wie üblich aufgearbeitet. Intermediär entsteht 2-Phenyl-5-hydroxyoctansäure, die nicht isoliert wird. Man erhält 2-Oxo-3-phenyl-6-propyl-tetrahydropyran.

Analog sind erhältlich:
2-Oxo-3-phenyl-6-butyl-tetrahydropyran
2-Oxo-3-phenyl-6-pentyl-tetrahydropyran
2-Oxo-3-phenyl-6-hexyl-tetrahydropyran
2-Oxo-3-phenyl-6-heptyl-tetrahydropyran
2-Oxo-3-phenyl-6-octyl-tetrahydropyran
2-Oxo-3-p-tolyl-6-propyl-tetrahydropyran
2-Oxo-3-p-tolyl-6-butyl-tetrahydropyran
2-Oxo-3-p-tolyl-6-pentyl-tetrahydropyran
2-Oxo-3-p-tolyl-6-hexyl-tetrahydropyran
2-Oxo-3-p-tolyl-6-heptyl-tetrahydropyran
2-Oxo-3-p-tolyl-6-octyl-tetrahydropyran
2-Oxo-3-p-ethylphenyl-propyl-tetrahydropyran
2-Oxo-3-p-ethylphenyl-butyl-tetrahydropyran
2-Oxo-3-p-ethylphenyl-pentyl-tetrahydropyran
2-Oxo-3-p-ethylphenyl-hexyl-tetrahydropyran
2-Oxo-3-p-ethylphenyl-heptyl-tetrahydropyran
2-Oxo-3-p-ethylphenyl-octyl-tetrahydropyran
2-Oxo-3-p-propylphenyl-propyl-tetrahydropyran
2-Oxo-3-p-propylphenyl-butyl-tetrahydropyran
2-Oxo-3-p-propylphenyl-pentyl-tetrahydropyran
2-Oxo-3-p-propylphenyl-hexyl-tetrahydropyran
2-Oxo-3-p-propylphenyl-heptyl-tetrahydropyran
2-Oxo-3-p-propylphenyl-octyl-tetrahydropyran
2-Oxo-3-p-butylphenyl-propyl-tetrahydropyran
2-Oxo-3-p-butylphenyl-butyl-tetrahydropyran
2-Oxo-3-p-butylphenyl-pentyl-tetrahydropyran
2-Oxo-3-p-butylphenyl-hexyl-tetrahydropyran
2-Oxo-3-p-butylphenyl-heptyl-tetrahydropyran
2-Oxo-3-p-butylphenyl-octyl-tetrahydropyran
2-Oxo-3-p-pentylphenyl-propyl-tetrahydropyran
2-Oxo-3-p-pentylphenyl-butyl-tetrahydropyran
2-Oxo-3-p-pentylphenyl-pentyl-tetrahydropyran
2-Oxo-3-p-pentylphenyl-hexyl-tetrahydropyran
2-Oxo-3-p-pentylphenyl-heptyl-tetrahydropyran
2-Oxo-3-p-pentylphenyl-octyl-tetrahydropyran

2-Oxo-3-p-hexylphenyl-propyl-tetrahydropyran
2-Oxo-3-p-hexylphenyl-butyl-tetrahydropyran
2-Oxo-3-p-hexylphenyl-pentyl-tetrahydropyran
2-Oxo-3-p-hexylphenyl-hexyl-tetrahydropyran
2-Oxo-3-p-hexylphenyl-heptyl-tetrahydropyran
2-Oxo-3-p-hexylphenyl-octyl-tetrahydropyran
2-Oxo-3-p-heptylphenyl-propyl-tetrahydropyran
2-Oxo-3-p-heptylphenyl-butyl-tetrahydropyran
2-Oxo-3-p-heptylphenyl-pentyl-tetrahydropyran
2-Oxo-3-p-heptylphenyl-hexyl-tetrahydropyran
2-Oxo-3-p-heptylphenyl-heptyl-tetrahydropyran
2-Oxo-3-p-heptylphenyl-octyl-tetrahydropyran
2-Oxo-3-p-octylphenyl-6-propyl-tetrahydropyran
2-Oxo-3-p-octylphenyl-6-butyl-tetrahydropyran
2-Oxo-3-p-octylphenyl-6-pentyl-tetrahydropyran
2-Oxo-3-p-octylphenyl-6-hexyl-tetrahydropyran
2-Oxo-3-p-octylphenyl-6-heptyl-tetrahydropyran
2-Oxo-3-p-octylphenyl-6-octyl-tetrahydropyran
2-Oxo-3-p-methoxyphenyl-6-propyl-tetrahydropyran
2-Oxo-3-p-methoxyphenyl-6-butyl-tetrahydropyran
2-Oxo-3-p-methoxyphenyl-6-pentyl-tetrahydropyran
2-Oxo-3-p-methoxyphenyl-6-hexyl-tetrahydropyran
2-Oxo-3-p-methoxyphenyl-6-heptyl-tetrahydropyran
2-Oxo-3-p-methoxyphenyl-6-octyl-tetrahydropyran
2-Oxo-3-p-ethoxyphenyl-6-propyl-tetrahydropyran
2-Oxo-3-p-ethoxyphenyl-6-butyl-tetrahydropyran
2-Oxo-3-p-ethoxyphenyl-6-pentyl-tetrahydropyran
2-Oxo-3-p-ethoxyphenyl-6-hexyl-tetrahydropyran
2-Oxo-3-p-ethoxyphenyl-6-heptyl-tetrahydropyran
2-Oxo-3-p-ethoxyphenyl-6-octyl-tetrahydropyran

Beispiel 7

Eine Lösung von 4,04 g 4-Hydroxymethyl-1-phenyl-nonan-1-ol-mono-p-toluolsulfonat (erhältlich durch Friedel-Crafts-Reaktion von 2-Pentylglutarsäureanhydrid mit Benzol zu 4-Benzoyl-2-pentylbuttersäure, $LiAlH_4$-Reduktion zu 4-Hydroxymethyl-1-phenyl-nonan-1-ol und Umsetzung mit p-Toluolsulfonylchlorid in Triethylamin/$CH_2Cl_2$) in 100 ml Phosphorsäure-hexamethyltriamid wird 24 Stunden auf 80° erwärmt. Man dampft ein, arbeitet wie üblich auf und erhält 2-Phenyl-5-pentyl-tetrahydropyran als Isomerengemisch, ölig. Isomerisierung von 2 g des Gemisches durch zweistündiges Erwärmen mit 0,19 K-tert.-Butylat in 20 ml Dimethylsulfoxid auf 80° führt zu trans-2-Phenyl-5-pentyl-tetrahydropyran

Analog sind erhältlich:
2-Phenyl-5-propyl-tetrahydropyran
2-Phenyl-5-butyl-tetrahydropyran
2-Phenyl-5-hexyl-tetrahydropyran
2-Phenyl-5-heptyl-tetrahydropyran
2-Phenyl-5-octyl-tetrahydropyran
2-p-Tolyl-5-propyl-tetrahydropyran
2-p-Tolyl-5-butyl-tetrahydropyran
2-p-Tolyl-5-pentyl-tetrahydropyran
2-p-Tolyl-5-hexyl-tetrahydropyran
2-p-Tolyl-5-heptyl-tetrahydropyran
2-p-Tolyl-5-octyl-tetrahydropyran
2-p-Ethylphenyl-5-propyl-tetrahydropyran
2-p-Ethylphenyl-5-butyl-tetrahydropyran
2-p-Ethylphenyl-5-pentyl-tetrahydropyran
2-p-Ethylphenyl-5-hexyl-tetrahydropyran
2-p-Ethylphenyl-5-heptyl-tetrahydropyran
2-p-Ethylphenyl-5-octyl-tetrahydropyran
2-p-Propylphenyl-5-propyl-tetrahydropyran
2-p-Propylphenyl-5-butyl-tetrahydropyran
2-p-Propylphenyl-5-pentyl-tetrahydropyran
2-p-Propylphenyl-5-hexyl-tetrahydropyran
2-p-Propylphenyl-5-heptyl-tetrahydropyran
2-p-Propylphenyl-5-octyl-tetrahydropyran
2-p-Butylphenyl-5-propyl-tetrahydropyran
2-p-Butylphenyl-5-butyl-tetrahydropyran
2-p-Butylphenyl-5-pentyl-tetrahydropyran
2-p-Butylphenyl-5-hexyl-tetrahydropyran
2-p-Butylphenyl-5-heptyl-tetrahydropyran
2-p-Butylphenyl-5-octyl-tetrahydropyran
2-p-Pentylphenyl-5-propyl-tetrahydropyran
2-p-Pentylphenyl-5-butyl-tetrahydropyran
2-p-Pentylphenyl-5-pentyl-tetrahydropyran
2-p-Pentylphenyl-5-hexyl-tetrahydropyran
2-p-Pentylphenyl-5-heptyl-tetrahydropyran, Kp. 210–215°/0,01 mbar
2-p-Pentylphenyl-5-octyl-tetrahydropyran
2-p-Hexylphenyl-5-propyl-tetrahydropyran
2-p-Hexylphenyl-5-butyl-tetrahydropyran
2-p-Hexylphenyl-5-pentyl-tetrahydropyran
2-p-Hexylphenyl-5-hexyl-tetrahydropyran
2-p-Hexylphenyl-5-heptyl-tetrahydropyran
2-p-Hexylphenyl-5-octyl-tetrahydropyran
2-p-Heptylphenyl-5-propyl-tetrahydropyran
2-p-Heptylphenyl-5-butyl-tetrahydropyran
2-p-Heptylphenyl-5-pentyl-tetrahydropyran
2-p-Heptylphenyl-5-hexyl-tetrahydropyran
2-p-Heptylphenyl-5-heptyl-tetrahydropyran
2-p-Heptylphenyl-5-octyl-tetrahydropyran
2-p-Octylphenyl-5-propyl-tetrahydropyran
2-p-Octylphenyl-5-butyl-tetrahydropyran
2-p-Octylphenyl-5-pentyl-tetrahydropyran
2-p-Octylphenyl-5-hexyl-tetrahydropyran
2-p-Octylphenyl-5-heptyl-tetrahydropyran
2-p-Octylphenyl-5-octyl-tetrahydropyran
2-(4-Biphenylyl)-5-propyl-tetrahydropyran
2-(4-Biphenylyl)-5-butyl-tetrahydropyran
2-(4-Biphenylyl)-5-pentyl-tetrahydropyran
2-(4-Biphenylyl)-5-hexyl-tetrahydropyran
2-(4-Biphenylyl)-5-heptyl-tetrahydropyran
2-(4-Biphenylyl)-5-octyl-tetrahydropyran
2-(4'-Methyl-4-biphenylyl)-5-propyl-tetrahydropyran
2-(4'-Methyl-4-biphenylyl)-5-butyl-tetrahydropyran
2-(4'-Methyl-4-biphenylyl)-5-pentyl-tetrahydropyran
2-(4'-Methyl-4-biphenylyl)-5-hexyl-tetrahydropyran
2-(4'-Methyl-4-biphenylyl)-5-heptyl-

tetrahydropyran
2-(4′-Methyl-4-biphenylyl)-5-octyl-tetrahydropyran
2-(4′-Ethyl-4-biphenylyl)-5-propyl-tetrahydropyran
2-(4′-Ethyl-4-biphenylyl)-5-butyl-tetrahydropyran
2-(4′-Ethyl-4-biphenylyl)-5-pentyl-tetrahydropyran
2-(4′-Ethyl-4-biphenylyl)-5-hexyl-tetrahydropyran
2-(4′-Ethyl-4-biphenylyl)-5-heptyl-tetrahydropyran
2-(4′-Ethyl-4-biphenylyl)-5-octyl-tetrahydropyran
2-(4′-Propyl-4-biphenylyl)-5-propyl-tetrahydropyran
2-(4′-Propyl-4-biphenylyl)-5-butyl-tetrahydropyran
2-(4′-Propyl-4-biphenylyl)-5-pentyl-tetrahydropyran
2-(4′-Propyl-4-biphenylyl)-5-hexyl-tetrahydropyran
2-(4′-Propyl-4-biphenylyl)-5-heptyl-tetrahydropyran
2-(4′-Propyl-4-biphenylyl)-5-octyl-tetrahydropyran
2-(4′-Butyl-4-biphenylyl)-5-propyl-tetrahydropyran
2-(4′-Butyl-4-biphenylyl)-5-butyl-tetrahydropyran
2-(4′-Butyl-4-biphenylyl)-5-pentyl-tetrahydropyran
2-(4′-Butyl-4-biphenylyl)-5-hexyl-tetrahydropyran
2-(4′-Butyl-4-biphenylyl)-5-heptyl-tetrahydropyran
2-(4′-Butyl-4-biphenylyl)-5-octyl-tetrahydropyran
2-(4′-Pentyl-4-biphenylyl)-5-propyl-tetrahydropyran
2-(4′-Pentyl-4-biphenylyl)-5-butyl-tetrahydropyran
2-(4′-Pentyl-4-biphenylyl)-5-pentyl-tetrahydropyran
2-(4′-Pentyl-4-biphenylyl)-5-hexyl-tetrahydropyran
2-(4′-Pentyl-4-biphenylyl)-5-heptyl-tetrahydropyran
2-(4′-Pentyl-4-biphenylyl)-5-octyl-tetrahydropyran
2-(4′-Hexyl-4-biphenylyl)-5-propyl-tetrahydropyran
2-(4′-Hexyl-4-biphenylyl)-5-butyl-tetrahydropyran
2-(4′-Hexyl-4-biphenylyl)-5-pentyl-tetrahydropyran
2-(4′-Hexyl-4-biphenylyl)-5-hexyl-tetrahydropyran
2-(4′-Hexyl-4-biphenylyl)-5-heptyl-tetrahydropyran
2-(4′-Hexyl-4-biphenylyl)-5-octyl-tetrahydropyran
2-(4′-Heptyl-4-biphenylyl)-5-propyl-tetrahydropyran
2-(4′-Heptyl-4-biphenylyl)-5-butyl-tetrahydropyran
2-(4′-Heptyl-4-biphenylyl)-5-pentyl-tetrahydropyran
2-(4′-Heptyl-4-biphenylyl)-5-hexyl-tetrahydropyran
2-(4′-Heptyl-4-biphenylyl)-5-heptyl-tetrahydropyran
2-(4′-Heptyl-4-biphenylyl)-5-octyl-tetrahydropyran
2-(4′-Octyl-4-biphenylyl)-5-propyl-tetrahydropyran
2-(4′-Octyl-4-biphenylyl)-5-butyl-tetrahydropyran
2-(4′-Octyl-4-biphenylyl)-5-pentyl-tetrahydropyran
2-(4′-Octyl-4-biphenylyl)-5-hexyl-tetrahydropyran
2-(4′-Octyl-4-biphenylyl)-5-heptyl-tetrahydropyran
2-(4′-Octyl-4-biphenylyl)-5-octyl-tetrahydropyran
2-(4′-Methoxy-4-biphenylyl)-5-propyl-tetrahydropyran
2-(4′-Methoxy-4-biphenylyl)-5-butyl-tetrahydropyran
2-(4′-Methoxy-4-biphenylyl)-5-pentyl-tetrahydropyran
2-(4′-Methoxy-4-biphenylyl)-5-hexyl-tetrahydropyran
2-(4′-Methoxy-4-biphenylyl)-5-heptyl-tetrahydropyran
2-(4′-Methoxy-4-biphenylyl)-5-octyl-tetrahydropyran
2-(4′-Ethoxy-4-biphenylyl)-5-propyl-tetrahydropyran
2-(4′-Ethoxy-4-biphenylyl)-5-butyl-tetrahydropyran
2-(4′-Ethoxy-4-biphenylyl)-5-pentyl-tetrahydropyran
2-(4′-Ethoxy-4-biphenylyl)-5-hexyl-tetrahydropyran
2-(4′-Ethoxy-4-biphenylyl)-5-heptyl-tetrahydropyran
2-(4′-Ethoxy-4-biphenylyl)-5-octyl-tetrahydropyran
2-(4′-(trans-4-Propylcyclohexyl)-4-biphenylyl)-5-propyl-tetrahydropyran
2-(4′-(trans-4-Propylcyclohexyl)-4-biphenylyl)-5-butyl-tetrahydropyran
2-(4′-(trans-4-Propylcyclohexyl)-4-biphenylyl)-5-pentyl-tetrahydropyran
2-(4′-(trans-4-Propylcyclohexyl)-4-biphenylyl)-5-hexyl-tetrahydropyran
2-(4′-(trans-4-Propylcyclohexyl)-4-biphenylyl)-5-heptyl-tetrahydropyran
2-(4′-(trans-4-Propylcyclohexyl)-4-biphenylyl)-5-octyl-tetrahydropyran
2-(4′-(trans-4-Butylcyclohexyl)-4-biphenylyl)-5-propyl-tetrahydropyran
2-(4′-(trans-4-Butylcyclohexyl)-4-biphenylyl)-5-butyl-tetrahydropyran
2-(4′-(trans-4-Butylcyclohexyl)-4-biphenylyl)-5-pentyl-tetrahydropyran
2-(4′-(trans-4-Butylcyclohexyl)-4-biphenylyl)-5-hexyl-tetrahydropyran
2-(4'-(trans-4-Butylcyclohexyl)-4-biphenylyl)-5-heptyl-tetrahydropyran
2-(4'-(trans-4-Butylcyclohexyl)-4-biphenylyl)-5-octyl-tetrahydropyran
2-(4'-(trans-4-Pentylcyclohexyl)-4-biphenylyl)-5-propyl-tetrahydropyran
2-(4'-(trans-4-Pentylcyclohexyl)-4-biphenylyl)-5-butyl-tetrahydropyran
2-(4'-(trans-4-Pentylcyclohexyl)-4-biphenylyl)-5-pentyl-tetrahydropyran
2-(4'-(trans-4-Pentylcyclohexyl)-4-biphenylyl)-5-hexyl-tetrahydropyran
2-(4'-(trans-4-Pentylcyclohexyl)-4-biphenylyl)-5-heptyl-tetrahydropyran
2-(4'-(trans-4-Pentylcyclohexyl)-4-biphenylyl)-5-octyl-tetrahydropyran
2-(4'-(trans-4-Hexylcyclohexyl)-4-biphenylyl)-5-propyl-tetrahydropyran
2-(4'-(trans-4-Hexylcyclohexyl)-4-biphenylyl)-5-butyl-tetrahydropyran
2-(4'-(trans-4-Hexylcyclohexyl)-4-biphenylyl)-5-pentyl-tetrahydropyran
2-(4'-(trans-4-Hexylcyclohexyl)-4-biphenylyl)-5-hexyl-tetrahydropyran
2-(4'-(trans-4-Hexylcyclohexyl)-4-biphenylyl)-5-heptyl-tetrahydropyran
2-(4'-(trans-4-Hexylcyclohexyl)-4-biphenylyl)-5-octyl-tetrahydropyran
2-(4'-(trans-4-Heptylcyclohexyl)-4-biphenylyl)-5-propyl-tetrahydropyran
2-(4'-(trans-4-Heptylcyclohexyl)-4-biphenylyl)-5-butyl-tetrahydropyran
2-(4'-(trans-4-Heptylcyclohexyl)-4-biphenylyl)-5-pentyl-tetrahydropyran
2-(4'-(trans-4-Heptylcyclohexyl)-4-biphenylyl)-5-hexyl-tetrahydropyran
2-(4'-(trans-4-Heptylcyclohexyl)-4-biphenylyl)-5-heptyl-tetrahydropyran
2-(4'-(trans-4-Heptylcyclohexyl)-4-biphenylyl)-5-octyl-tetrahydropyran

Beispiel 8

Man löst 24,7 g 2-p-Aminophenyl-5-pentyl-tetrahydropyran (erhältlich z.B. durch Nitrierung von 2-Phenyl-5-pentyl-tetrahydropyran und anschliessende Hydrierung des erhaltenen 2-p-Nitrophenyl-5-pentyl-tetrahydropyrans) in einem Gemisch von 25 ml konzentrierter Salzsäure und 75 ml Wasser und diazotiert bei 3–6° mit einer Lösung von 8 g $NaNO_2$ in 15 ml Wasser. Die so erhaltene Diazoniumsalzlösung wird einer auf 60–70° erwärmten $Cu_2(CN)_2$-Lösung (hergestellt durch Erwärmen von 25 g Kupfersulfat mit 28 g KCN in 100 ml Wasser) innerhalb 15 Minuten zugesetzt. Man erwärmt noch 20 Minuten auf 100°, kühlt ab und erhält nach üblicher Aufarbeitung 2-p-Cyanphenyl-5-pentyl-tetrahydropyran.

Analog sind erhältlich:
2-p-Cyanphenyl-5-methyl-tetrahydropyran
2-p-Cyanphenyl-5-ethyl-tetrahydropyran
2-p-Cyanphenyl-5-propyl-tetrahydropyran
2-p-Cyanphenyl-5-butyl-tetrahydropyran
2-p-Cyanphenyl-5-hexyl-tetrahydropyran
2-p-Cyanphenyl-5-heptyl-tetrahydropyran
2-p-Cyanphenyl-5-octyl-tetrahydropyran
2-p-Cyanphenyl-5-nonyl-tetrahydropyran
2-p-Cyanphenyl-5-decyl-tetrahydropyran
2-Oxo-3-propyl-6-p-cyanphenyl-tetrahydropyran
2-Oxo-3-butyl-6-p-cyanphenyl-tetrahydropyran
2-Oxo-3-pentyl-6-p-cyanphenyl-tetrahydropyran
2-Oxo-3-hexyl-6-p-cyanphenyl-tetrahydropyran
2-Oxo-3-heptyl-6-p-cyanphenyl-tetrahydropyran
2-Oxo-3-octyl-6-p-cyanphenyl-tetrahydropyran
2-(4'-Cyan-4-biphenylyl)-5-propyl-tetrahydropyran
2-(4'-Cyan-4-biphenylyl)-5-butyl-tetrahydropyran
2-(4'-Cyan-4-biphenylyl)-5-pentyl-tetrahydropyran
2-(4'-Cyan-4-biphenylyl)-5-hexyl-tetrahydropyran
2-(4'-Cyan-4-biphenylyl)-5-heptyl-tetrahydropyran
1-(4'-Cyan-4-biphenylyl)-5-octyl-tetrahydropyran
2-Methyl-5-p-cyanphenyl-tetrahydropyran
2-Ethyl-5-p-cyanphenyl-tetrahydropyran
2-Propyl-5-p-cyanphenyl-tetrahydropyran
2-Butyl-5-p-cyanphenyl-tetrahydropyran
2-Pentyl-5-p-cyanphenyl-tetrahydropyran
2-Hexyl-5-p-cyanphenyl-tetrahydropyran
2-Heptyl-5-p-cyanphenyl-tetrahydropyran
2-Octyl-5-p-cyanphenyl-tetrahydropyran
2-Nonyl-5-p-cyanphenyl-tetrahydropyran
2-Decyl-5-p-cyanphenyl-tetrahydropyran
2-Oxo-3-p-cyanphenyl-6-propyl-tetrahydropyran
2-Oxo-3-p-cyanphenyl-6-butyl-tetrahydropyran
2-Oxo-3-p-cyanphenyl-6-pentyl-tetrahydropyran
2-Oxo-3-p-cyanphenyl-6-hexyl-tetrahydropyran
2-Oxo-3-p-cyanphenyl-6-heptyl-tetrahydropyran
2-Oxo-3-p-cyanphenyl-6-octyl-tetrahydropyran
2-Propyl-5-(4'-cyan-4-biphenylyl)-tetrahydropyran
2-Butyl-5-(4'-cyan-4-biphenylyl)-tetrahydropyran
2-Pentyl-5-(4'-cyan-4-biphenylyl)-tetrahydropyran
2-Hexyl-5-(4'-cyan-4-biphenylyl)-tetrahydropyran
2-Heptyl-5-(4'-cyan-4-biphenylyl)-tetrahydropyran
2-Octyl-5-(4'-cyan-4-biphenylyl)-tetrahydropyran

Beispiel 9

Man arbeitet analog Beispiel 8, verwendet jedoch an Stelle der $Cu_2(CN)_2$-Lösung eine (aus 25 g Kupfersulfat und 25 g NaCl in 100 ml Wasser mit $SO_2$ hergestellte) schwach siedende $Cu_2Cl_2$-Lösung und erhält 2-p-Chlorphenyl-5-pentyl-tetrahydropyran.

Analog sind erhältlich:
2-p-Chlorphenyl-5-propyl-tetrahydropyran
2-p-Chlorphenyl-5-butyl-tetrahydropyran
2-p-Chlorphenyl-5-hexyl-tetrahydropyran
2-p-Chlorphenyl-5-heptyl-tetrahydropyran
2-p-Chlorphenyl-5-octyl-tetrahydropyran

2-Oxo-3-propyl-6-p-chlorphenyl-tetrahydropyran
2-Oxo-3-butyl-6-p-chlorphenyl-tetrahydropyran
2-Oxo-3-pentyl-6-p-chlorphenyl-tetrahydropyran
2-Oxo-3-hexyl-6-p-chlorphenyl-tetrahydropyran
2-Oxo-3-heptyl-6-p-chlorphenyl-tetrahydropyran
2-Oxo-3-octyl-6-p-chlorphenyl-tetrahydropyran
2-(4'-Chlor-4-biphenylyl)-5-propyl-tetrahydropyran
2-(4'-Chlor-4-biphenylyl)-5-butyl-tetrahydropyran
2-(4'-Chlor-4-biphenylyl)-5-pentyl-tetrahydropyran
2-(4'-Chlor-4-biphenylyl)-5-hexyl-tetrahydropyran
2-(4'-Chlor-4-biphenylyl)-5-heptyl-tetrahydropyran
2-(4'-Chlor-4-biphenylyl)-5-octyl-tetrahydropyran
2-Propyl-5-p-chlorphenyl-tetrahydropyran
2-Butyl-5-p-chlorphenyl-tetrahydropyran
2-Pentyl-5-p-chlorphenyl-tetrahydropyran
2-Hexyl-5-p-chlorphenyl-tetrahydropyran
2-Heptyl-5-p-chlorphenyl-tetrahydropyran
2-Octyl-5-p-chlorphenyl-tetrahydropyran
2-Oxo-3-p-chlorphenyl-6-propyl-tetrahydropyran
2-Oxo-3-p-chlorphenyl-6-butyl-tetrahydropyran
2-Oxo-3-p-chlorphenyl-6-pentyl-tetrahydropyran
2-Oxo-3-p-chlorphenyl-6-hexyl-tetrahydropyran
2-Oxo-3-p-chlorphenyl-6-heptyl-tetrahydropyran
2-Oxo-3-p-chlorphenyl-6-octyl-tetrahydropyran
2-Propyl-5-(4'chlor-4-biphenylyl)-tetrahydropyran
2-Butyl-5-(4'chlor-4-biphenylyl)-tetrahydropyran
2-Pentyl-5-(4'chlor-4-biphenylyl)-tetrahydropyran
2-Hexyl-5-(4'chlor-4-biphenylyl)-tetrahydropyran
2-Heptyl-5-(4'chlor-4-biphenylyl)-tetrahydropyran
2-Octyl-5-(4'chlor-4-biphenylyl)-tetrahydropyran

Beispiel 10

Man arbeitet analog Beispiel 8, verwendet jedoch an Stelle der $Cu_2(CN)_2$-Lösung eine (aus 25 g Kupfersulfat, 8 g Kupferpulver, 52 g KBr, 6,4 ml konzentrierter $H_2SO_4$ und 400 ml Wasser durch 3std. Kochen hergestellte) siedende $Cu_2Br_2$-Lösung und erhält 2-p-Bromphenyl-5-pentyl-tetrahydropyran

Analog sind erhältlich:
2-p-Bromphenyl-5-propyl-tetrahydropyran
2-p-Bromphenyl-5-butyl-tetrahydropyran
2-p-Bromphenyl-5-hexyl-tetrahydropyran
2-p-Bromphenyl-5-heptyl-tetrahydropyran
2-p-Bromphenyl-5-octyl-tetrahydropyran
2-Oxo-3-propyl-6-p-bromphenyl-tetrahydropyran
2-Oxo-3-butyl-6-p-bromphenyl-tetrahydropyran
2-Oxo-3-pentyl-6-p-bromphenyl-tetrahydropyran
2-Oxo-3-hexyl-6-p-bromphenyl-tetrahydropyran
2-Oxo-3-heptyl-6-p-bromphenyl-tetrahydropyran
2-Oxo-3-octyl-6-p-bromphenyl-tetrahydropyran
2-(4'-Brom-4-biphenylyl)-5-propyl-tetrahydropyran
2-(4'-Brom-4-biphenylyl)-5-butyl-tetrahydropyran
2-(4'-Brom-4-biphenylyl)-5-pentyl-tetrahydropyran
2-(4'-Brom-4-biphenylyl)-5-hexyl-tetrahydropyran
2-(4'-Brom-4-biphenylyl)-5-heptyl-tetrahydropyran
2-(4'-Brom-4-biphenylyl)-5-octyl-tetrahydropyran
2-Propyl-5-p-bromphenyl-tetrahydropyran
2-Butyl-5-p-bromphenyl-tetrahydropyran
2-Pentyl-5-p-bromphenyl-tetrahydropyran
2-Hexyl-5-p-bromphenyl-tetrahydropyran
2-Heptyl-5-p-bromphenyl-tetrahydropyran
2-Octyl-5-p-bromphenyl-tetrahydropyran
2-Oxo-3-p-bromphenyl-6-propyl-tetrahydropyran
2-Oxo-3-p-bromphenyl-6-butyl-tetrahydropyran
2-Oxo-3-p-bromphenyl-6-pentyl-tetrahydropyran
2-Oxo-3-p-bromphenyl-6-hexyl-tetrahydropyran
2-Oxo-3-p-bromphenyl-6-heptyl-tetrahydropyran
2-Oxo-3-p-bromphenyl-6-octyl-tetrahydropyran
2-Propyl-5-(4'-brom-4-biphenylyl)-tetrahydropyran
2-Butyl-5-(4'-brom-4-biphenylyl)-tetrahydropyran
2-Pentyl-5-(4'-brom-4-biphenylyl)-tetrahydropyran
2-Hexyl-5-(4'-brom-4-biphenylyl)-tetrahydropyran
2-Heptyl-5-(4'-brom-4-biphenylyl)-tetrahydropyran
2-Octyl-5-(4'-brom-4-biphenylyl)-tetrahydropyran

Beispiel 11

Man löst 24,7 g 2-p-Aminophenyl-5-pentyl-tetrahydropyran in einem Gemisch von 25 ml konzentrierter Salzsäure und 75 ml Wasser und diazotiert bei 3–6° mit einer Lösung von 8 g $NaNO_2$ in 15 ml Wasser. Anschliessend gibt man 24 g 40%ige Tetrafluorborsäure zu, filtriert das ausgefallene Diazoniumtetrafluorborat ab, wäscht mit Wasser und trocknet. Das Diazoniumsalz wird auf etwa 150° erhitzt, der Rückstand wie üblich aufgearbeitet. Man erhält 2-p-Fluorphenyl-5-pentyl-tetrahydropyran

Analog erhält man:
2-p-Fluorphenyl-5-propyl-tetrahydropyran
2-p-Fluorphenyl-5-butyl-tetrahydropyran
2-p-Fluorphenyl-5-hexyl-tetrahydropyran
2-p-Fluorphenyl-5-heptyl-tetrahydropyran
2-p-Fluorphenyl-5-octyl-tetrahydropyran
2-Oxo-3-propyl-6-p-fluorphenyl-tetrahydropyran
2-Oxo-3-butyl-6-p-fluorphenyl-tetrahydropyran
2-Oxo-3-pentyl-6-p-fluorphenyl-tetrahydropyran

2-Oxo-3-hexyl-6-p-fluorphenyl-tetrahydropyran
2-Oxo-3-heptyl-6-p-fluorphenyl-tetrahydropyran
2-Oxo-3-octyl-6-p-fluorphenyl-tetrahydropyran
2-(4′-Fluor-4-biphenylyl)-5-propyl-tetrahydropyran
2-(4′-Fluor-4-biphenylyl)-5-butyl-tetrahydropyran
2-(4′-Fluor-4-biphenylyl)-5-pentyl-tetrahydropyran
2-(4′-Fluor-4-biphenylyl)-5-hexyl-tetrahydropyran
2-(4′-Fluor-4-biphenylyl)-5-heptyl-tetrahydropyran
2-(4′-Fluor-4-biphenylyl)-5-octyl-tetrahydropyran
2-Propyl-5-p-fluorphenyl-tetrahydropyran
2-Butyl-5-p-fluorphenyl-tetrahydropyran
2-Pentyl-5-p-fluorphenyl-tetrahydropyran
2-Hexyl-5-p-fluorphenyl-tetrahydropyran
2-Heptyl-5-p-fluorphenyl-tetrahydropyran
2-Octyl-5-p-fluorphenyl-tetrahydropyran
2-Oxo-3-p-fluorphenyl-6-propyl-tetrahydropyran
2-Oxo-3-p-fluorphenyl-6-butyl-tetrahydropyran
2-Oxo-3-p-fluorphenyl-6-pentyl-tetrahydropyran
2-Oxo-3-p-fluorphenyl-6-hexyl-tetrahydropyran
2-Oxo-3-p-fluorphenyl-6-heptyl-tetrahydropyran
2-Oxo-3-p-fluorphenyl-6-octyl-tetrahydropyran
2-Propyl-5-(4′-fluor-4-biphenylyl)-tetrahydropyran
2-Butyl-5-(4′-fluor-4-biphenylyl)-tetrahydropyran
2-Pentyl-5-(4′-fluor-4-biphenylyl)-tetrahydropyran
2-Hexyl-5-(4′-fluor-4-biphenylyl)-tetrahydropyran
2-Heptyl-5-(4′-fluor-4-biphenylyl)-tetrahydropyran
2-Octyl-5-(4′-fluor-4-biphenylyl)-tetrahydropyran.

Beispiel 12

Ein Gemisch von 2,94 g 2-Fluor-4-(5-pentyl-tetrahydropyran-2-yl)-benzoesäure [erhältlich durch Reaktion von 2-m-Fluorphenyl-5-pentyl-tetrahydropyran mit Acetylchlorid/$ZnCl_2$ zu 2-(4-Acetyl-3-fluorphenyl)-5-pentyl-tetrahydropyran und Abbau mit $Br_2$/KOH], 1,42 g trans-4-Propylcyclohexanol und 2,06 g Dicyclohexylcarbodiimid in 50 ml Diethylether wird 6 Stunden gekocht. Nach dem Abkühlen filtriert man, arbeitet wie üblich auf und erhält 2-Fluor-4-(5-pentyl-tetrahydropyran-2-yl)-benzoesäure-(trans-4-propyl-cyclohexylester).

Analog erhält man:
4-(5-Propyl-tetrahydropyran-2-yl)-benzoesäure-(trans-4-methoxymethyl-cyclohexylester)
4-(5-Pentyl-tetrahydropyran-2-yl)-benzoesäure-(p-2-methoxyethoxy-phenylester)
4-(5-Pentyl-tetrahydropyran-2-yl)-benzoesäure-(2,3,5,6-tetrafluorphenylester)
4-(5-Pentyl-tetrahydropyran-2-yl)-benzoesäure-(1-propyl-4-piperidylester)
4-(5-Pentyl-tetrahydropyran-2-yl)-benzoesäure-(1,4-bicyclo(2,2,2)octylester)
4-(5-Pentyl-tetrahydropyran-2-yl)-benzoesäure-(5-methoxypyrimidin-2-ylester).

Beispiel 13

Man kocht 27,6 g p-(5-Pentyl-tetrahydropyran-2-yl)-benzoesäure (erhältlich durch Reaktion von 2-Phenyl-5-pentyl-tetrahydropyran mit Acetylchlorid/$AlCl_3$ zu 2-p-Acetylphenyl-5-pentyl-tetrahydropyran und Abbau mit Brom/KOH) 1 Stunde mit 24 g $SOCl_2$, dampft ein, löst das erhaltene rohe Säurechlorid in 150 ml Toluol, versetzt mit 8 ml Pyridin und 16,6 g trans-4-Pentyl-cyclohexanol und kocht zwei Stunden. Nach Abkühlen und üblicher Aufarbeitung erhält man p-(5-Pentyl-tetrahydropyran-2-yl)-benzoesäure-(trans-4-pentylcyclohexylester).

Beispiel 14

Ein Gemisch von 1,2 g 2-Propylpropan-1,3-diol, 2,2 g 5-Formyl-2-p-methoxyphenyl-tetrahydropyran [erhältlich durch Reaktion von Ethylendioxymethyl-malonsäurediethylester mit ω-Brom-p-methoxy-propiophenon zu 2-Ethylendioxymethyl-2-(3-oxo-3-p-methoxyphenylpropyl)-malonsäurediethylester, $NaBH_4$-Reduktion zum Alkohol, Verseifung, Decarboxylierung und Lactonisierung zu 2-Oxo-3-ethylendioxymethyl-6-p-methoxyphenyl-tetrahydropyran, Reduktion mit $LiAlH_4$ zum Diol und Cyclisierung mit p-Toluolsulfonsäure unter gleichzeitiger Spaltung des Acetals], 0,01 g p-Toluolsulfonsäure und 15 ml Toluol wird am Wasserabscheider 3 Stunden gekocht, abgekühlt, mit Wasser gewaschen und eingedampft. Man erhält 2-p-Methoxyphenyl-5-(trans-5-propyl-1,3-dioxan-2-yl)-tetrahydropyran.

Analog erhält man:
2-p-Methoxyphenyl-5-(trans-5-butyl-1,3-dioxan-2-yl)-tetrahydropyran
2-p-Methoxyphenyl-5-(trans-5-pentyl-1,3-dioxan-2-yl)-tetrahydropyran
2-p-Methoxyphenyl-5-(trans-5-hexyl-1,3-dioxan-2-yl)-tetrahydropyran
2-p-Methoxyphenyl-5-(trans-5-heptyl-1,3-dioxan-2-yl)-tetrahydropyran
2-p-Methoxyphenyl-5-(trans-5-octyl-1,3-dioxan-2-yl)-tetrahydropyran

Beispiel 15

Eine Lösung von 29,3 g 2-Fluor-4-(5-pentyl-tetrahydropyran-2-yl)-benzamid (erhältlich aus dem Säurechlorid und $NH_3$) in 500 ml DMF wird bei 50° unter Rühren tropfenweise mit 65 g $POCl_3$ versetzt. Nach weiterem einstündigem Rühren giesst man auf Eis, arbeitet wie üblich auf und erhält 2-Fluor-4-(5-pentyl-tetrahydropyran-2-yl)-benzonitril.

Analog erhält man:
2-Fluor-4-(5-propyl-tetrahydropyran-2-yl)-benzonitril
2-Fluor-4-(5-butyl-tetrahydropyran-2-yl)-benzonitril
2-Fluor-4-(5-pentyl-tetrahydropyran-2-yl)-benzonitril

2-Fluor-4-(5-hexyl-tetrahydropyran-2-yl) -benzonitril
2-Fluor-4-(5-heptyl-tetrahydropyran-2-yl) -benzonitril
2-Fluor-4-(5-octyl-tetrahydropyran-2-yl) -benzonitril

Beispiel 16

Eine Lösung von 29,5 g p-(5-Pentyl-tetrahydropyran-2-yl)-benzoylchlorid und 8 g Sulfamid in 500 ml Tetramethylensulfon wird 4 Stunden auf 120° erhitzt, eingedampft und wie üblich aufgearbeitet. Man erhält 2-p-Cyanphenyl-5-pentyl-tetrahydropyran.

Analog sind die übrigen in Beispiel 8 angegebenen Verbindungen erhältlich.

Beispiel 17

Ein Gemisch von 24,8 g 2-p-Hydroxyphenyl-5-pentyl-tetrahydropyran (erhältlich durch Diazotierung von 2-p-Aminophenyl-5-pentyl-tetrahydropyran und Hydrolyse), 6,9 g $K_2CO_3$, 25 g Hexyljodid und 250 ml DMF wird unter Rühren 16 Stunden auf 80° erhitzt, dann abgekühlt und wie üblich aufgearbeitet. Man erhält 2-p-Hexoxyphenyl-5-pentyl-tetrahydropyran.

Analog erhält man:
2-p-Butoxyphenyl-5-propyl-tetrahydropyran
2-p-Butoxyphenyl-5-butyl-tetrahydropyran
2-p-Butoxyphenyl-5-pentyl-tetrahydropyran
2-p-Butoxyphenyl-5-hexyl-tetrahydropyran
2-p-Butoxyphenyl-5-heptyl-tetrahydropyran
2-p-Butoxyphenyl-5-octyl-tetrahydropyran
2-p-Pentoxyphenyl-5-propyl-tetrahydropyran
2-p-Pentoxyphenyl-5-butyl-tetrahydropyran
2-p-Pentoxyphenyl-5-pentyl-tetrahydropyran
2-p-Pentoxyphenyl-5-hexyl-tetrahydropyran
2-p-Pentoxyphenyl-5-heptyl-tetrahydropyran
2-p-Pentoxyphenyl-5-octyl-tetrahydropyran
2-p-Hexoxyphenyl-5-propyl-tetrahydropyran
2-p-Hexoxyphenyl-5-butyl-tetrahydropyran
2-p-Hexoxyphenyl-5-hexyl-tetrahydropyran
2-p-Hexoxyphenyl-5-heptyl-tetrahydropyran
2-p-Hexoxyphenyl-5-octyl-tetrahydropyran

Beispiel 18

Ein Gemisch aus 31,1 g 2-p-Bromphenyl-5-pentyl-tetrahydropyran, 10 g $Cu_2(CN)_2$, 120 ml Pyridin und 60 ml N-Methylpyrrolidon wird 2 Stunden auf 150° erhitzt. Man kühlt ab, gibt eine Lösung von 120 g $FeCl_3 \cdot 6\ H_2O$ in 600 ml 20%iger Salzsäure hinzu, erwärmt 1,5 Stunden unter Rühren auf 70°, arbeitet wie üblich auf und erhält 2-p-Cyanphenyl-5-pentyl-tetrahydropyran.

Analog sind die übrigen in Beispiel 8 angegebenen Verbindungen erhältlich.

Es folgen Beispiele für erfindungsgemässe Dielektrika, die mindestens eine Verbindung der Formel I enthalten:

Beispiel A

Ein Gemisch aus
15% 2-p-Cyanphenyl-5-propyl-tetrahydropyran
27% 2-p-Ethylphenyl-5-propyl-tetrahydropyran
10% trans-1-p-Ethoxyphenyl-4-propylcyclohexan
7% 4-Cyn-4'-(trans-4-pentylcyclohexyl)-biphenyl
9% 1-(trans-4-Propylcyclohexyl)-2-(p-trans-4-propylcyclohexyl-phenyl)-ethan
8% 4-Ethyl-4'-(trans-4-pentylcyclohexyl)-biphenyl
10% 4-(trans-4-Pentylcyclohexyl)-4'-(trans-4-propylcyclohexyl)-biphenyl
14% trans-4-Butylcyclohexancarbonsäure-(p-trans-4-propylcyclohexylphenylester
zeigt K. 85°

Beispiel B

Ein Gemisch aus
17% 2-p-Cyanphenyl-5-propyl-tetrahydropyran
23% p-trans-4-Butylcyclohexyl-benzonitril
14% 4-Ethyl-4'-cyan-biphenyl
26% 4-Butyl-4'-cyan-biphenyl
8% 4,4'-Bis-(trans-4-propylcyclohexyl)-biphenyl
12% p-Pentylbenzoesäure-(p-cyanphenylester)
zeigt K. 61°.

Beispiel C (Mischung mit negativer DK-Anisotropie)

Ein Gemisch aus
25% 2-p-Ethoxyphenyl-5-propyl-tetrahydropyran
25% trans-1-p-Butoxyphenyl-4-propyl-cyclohexan
15% trans-4-Pentylcyclohexancarbonsäure-(p-pentylphenylester)
15% trans-4-Propylcyclohexancarbonsäure-(p-ethoxyphenylester)
10% 4-(trans-4-Pentylcyclohexyl)-4'-(trans-4-propyl-cyclohexyl)-biphenyl
10% p-trans-4-Propylcyclohexyl-benzoesäure-(4-butyl-2-cyanphenylester)
zeigt K. 73°.

Beispiel D (Matrix für dichroitische Farbstoffe)

Ein Gemisch aus
15% 2-p-Cyanphenyl-5-propyl-tetrahydropyran
14% 2-p-Cyanphenyl-5-butyl-tetrahydropyran
18% p-trans-4-Propylcyclohexyl-benzonitril
25% p-trans-4-Butylcyclohexyl-benzonitril
7% 4-Cyan-4'-(trans-4-pentylcyclohexyl)-biphenyl
6% 4-p-Cyanphenyl-4'-pentyl-biphenyl
7% 4-(trans-4-Pentylcyclohexyl)-4'-(trans-4-propylcyclohexyl)-biphenyl
8% trans-4-Butylcyclohexancarbonsäure-(p-trans-4-propylcyclohexylphenylester)
zeigt K. 86°.

Beispiel E

In einem Gemisch aus

98% Matrix nach Beispiel D
2% 1,5-Dihydroxy-2,6-bis-(trans-4-propylcyclohexyl)-4,8-diamino-anthrachinon
zeigt der Farbstoff einen Ordnungsgrad von 0,75.

Beispiel F

Ein Gemisch aus
8% 2-Oxo-3-heptyl-6-p-pentylphenyl-tetrahydropyran
21% trans-1-p-Ethoxyphenyl-4-propyl-cyclohexan
18% trans-1-p-Butoxyphenyl-4-propyl-cyclohexan
41% Buttersäure-(p-trans-4-propylcyclohexyl-phenylester)
12% 4-(trans-4-Pentylcyclohexyl)-4′-(trans-4-propyl-cyclohexyl)-biphenyl
zeigt F. −10°, K. 59°.

**Patentansprüche**

1. Tetrahydropyranderivate der Formel I

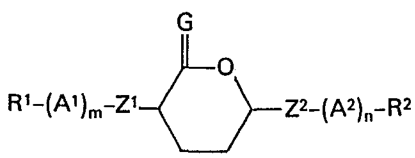

I

worin
G $H_2$ oder $=O$,
$R^1$ und $R^2$ jeweils eine Alkylgruppe mit 1–10 C-Atomen, worin auch eine oder zwei $CH_2$-Gruppen durch O-Atome ersetzt sein können, F, Cl, Br oder CN,
$R^2$ auch H,
$A^1$ und $A^2$ jeweils unsubstituierte oder durch 1–4 F-Atome substituierte 1,4-Phenylen-, 1,4-Cyclohexylen-, 1,3-Dioxan-2,5-diyl-, Piperidin-1,4-diyl-, 1,4-Bicyclo[2,2,2]-octylen- oder Pyrimidin-2,5-diylgruppen,
$Z^1$ und $Z^2$ jeweils $-CO-O-$, $-O-CO-$, $-CH_2CH_2-$, $-OCH_2-$, $-CH_2O-$ oder eine Einfachbindung,
m und n jeweils 0, 1, 2 oder 3 bedeuten,
(m + n) jedoch mindestens 1 und höchstens 3 sind,
wobei für m = 2 oder 3 die Gruppen $A^1$ und für n = 2 oder 3 die Gruppen $A^2$ jeweils gleich oder voneinander verschieden sein können,
sowie die Säureadditionssalze der basischen unter diesen Verbindungen.

2. Verfahren zur Herstellung von Tetrahydropyranen der Formel I nach Anspruch 1, dadurch gekennzeichnet, dass man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,
oder dass man eine Verbindung der Formel II

$$R^1-(A^1)_m-Z^1-CH(CGOH)-CH_2CH_2-CHOH-Z^2-(A^2)_n-R^2 \quad II$$

worin

G, $R^1$, $R^2$, $A^1$, $A^2$, $Z^1$, $Z^2$, m und n die angegebenen Bedeutungen haben oder eines ihrer reaktionsfähigen Derivate cyclisiert,
oder dass man eine Verbindung der Formel III

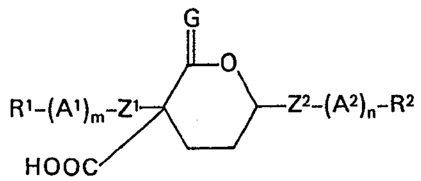

III

worin
G, $R^1$, $R^2$, $A^1$, $A^2$, $Z^1$, $Z^2$, m und n die angegebenen Bedeutungen haben decarboxyliert,
oder dass man zur Herstellung von Verbindungen der Formel I, worin $R^1$ und/oder $R^2$ F, Cl, Br oder CN bedeuten, in einem entsprechenden Diazoniumsalz die Diazoniumgruppe durch F, Cl, Br oder CN ersetzt,
oder dass man zur Herstellung von Estern der Formel I (worin $Z^1$ und/oder $Z^2$ $-CO-O-$ oder $-O-CO-$ bedeuten) eine entsprechende Carbonsäure oder eines ihrer reaktionsfähigen Derivate mit einem entsprechenden Alkohol oder einem seiner reaktionsfähigen Derivate umsetzt,
oder dass man zur Herstellung von Dioxanderivaten der Formel I (worin $A^1$ und/oder $A^2$ 1,3-Dioxan-2,5-diyl bedeuten) einen entsprechenden Aldehyd mit einem entsprechenden Diol umsetzt,
oder dass man zur Herstellung von Nitrilen der Formel I (worin $R^1$ und/oder $R^2$ CN bedeuten) ein entsprechendes Carbonsäureamid dehydratisiert oder ein entsprechendes Carbonsäurehalogenid mit Sulfamid umsetzt,
oder dass man zur Herstellung von Ethern der Formel I (worin $R^1$ und/oder $R^2$ Alkylketten bedeuten, worin eine oder zwei $CH_2$-Gruppen durch O-Atome ersetzt sind, und/oder $Z^1$ und/oder $Z^2$ $-OCH_2-$ oder $-CH_2O-$ Gruppen sind) eine entsprechende Hydroxyverbindung verethert,
und/oder dass man gegebenenfalls eine Chlor- oder Bromverbindung der Formel I (worin $R^1$ und/oder $R^2$ Cl oder Br bedeuten) mit einem Cyanid umsetzt,
und/oder dass man gegebenenfalls eine Base der Formel I durch Behandeln mit einer Säure in eines ihrer Säureadditionssalze umwandelt,
oder dass man gegebenenfalls eine Verbindung der Formel I aus einem ihrer Säureadditionssalze durch Behandeln mit einer Base freisetzt.

3. Verwendung der Verbindungen der Formel I nach Anspruch I als Komponenten flüssigkristalliner Dielektrika für elektrooptische Anzeigeelemente.

4. Flüssigkristallines Dielektrikum für elektrooptische Anzeigeelemente mit mindestens zwei flüssigkristallinen Komponenten, dadurch gekennzeichnet, dass mindestens eine Komponente eine Verbindung der Formel I nach Anspruch 1 ist.

5. Elektrooptisches Anzeigeelement, dadurch gekennzeichnet, dass es ein Dielektrikum nach Anspruch 4 enthält.

**Claims**

1. Tetrahydropyran derivatives of the formula I

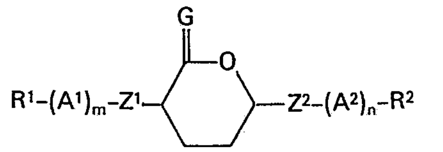

I

in which G is $H_2$ or =O, $R^1$ and $R^2$ are each an alkyl group having 1–10 C atoms, it also being possible for one or two $CH_2$ groups to be replaced by O atoms, or are F, Cl, Br or CN, $R^2$ is also H, $A^1$ and $A^2$ are each 1,4-phenylene, 1,4-cyclohexylene, 1,3-dioxane-2,5-diyl, piperidine-1,4-diyl, 1,4-bicyclo[2.2.2]octylene or pyrimidine-2,5-diyl groups which are unsubstituted or substituted by 1–4 F atoms, $Z^1$ and $Z^2$ are each -CO-O-, -O-CO-, $-CH_2CH_2-$, $-OCH_2-$, $-CH_2O-$, or a single bond, m and n are each 0, 1, 2 or 3, but (m + n) is at least 1 and at most 3, it being possible, when m is 2 or 3, for the groups $A^1$ and, when n is 2 or 3, for the groups $A^2$ each to be identical or different from one another, and the acid addition salts of those among these compounds which are basic.

2. Process for the preparation of tetrahydropyrans of the formula I according to Claim 1, characterised in that a compound which otherwise corresponds to formula I but, in place of H atoms, contains one or more reducible groups and/or C–C bonds, it treated with a reducing agent, or in that a compound of the formula II

$R^1-(A^1)_m-Z^1-CH(CGOH)-CH_2CH_2-CHOH-Z^2-(A^2)_n-R^2$ II

in which G, $R^1$, $R^2$, $A^1$, $A^2$, $Z^1$, $Z^2$, m and n have the indicated meanings, or one of its reactive derivatives is cyclised, or in that a compound of the formula III

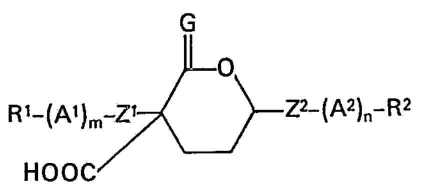

III

in which G, $R^1$, $R^2$, $A^1$, $A^2$, $Z^1$, $Z^2$, m and n have the indicated meanings, is decarboxylated, or in that, for the preparation of compounds of the formula I in which $R^1$ and/or $R^2$ are F, Cl, Br or CN, the diazonium group in an appropriate diazonium salt is replaced by F, Cl, Br or CN, or in that, for the preparation of esters of the formula I (in which $Z^1$ and/or $Z^2$ is -CO-O- or -O-CO-), an appropriate carboxylic acid or one of its reactive derivatives is reacted with an appropriate alcohol or one of its reactive derivatives, or in that, for the preparation of dioxane derivatives of the formula I (in which $A^1$ and/or $A^2$ is 1,3-dioxane-2,5-diyl), an appropriate aldehyde is reacted with an appropriate diol, or in that, for the preparation of nitriles of the formula I (in which $R^1$ and/or $R^2$ is CN), an appropriate carboxamide is dehydrated or an appropriate carbonyl halide is reacted with sulfamide, or in that, for the preparation of ethers of the formula I (in which $R^1$ and/or $R^2$ are alkyl chains, in which one or two $CH_2$ groups are replaced by O atoms, and/or $Z^1$ and/or $Z^2$ are $-OCH_2-$ or $-CH_2O-$ groups), an appropriate hydroxy compound is etherified, and/or in that, where appropriate, a chloro or bromo compound of the formula I (in which $R^1$ and/or $R^2$ is Cl or Br) is reacted with a cyanide, and/or in that, where appropriate, a base of the formula I is converted, by treatment with an acid, into one of its acid addition salts, or in that, where appropriate, a compound of the formula I is liberated from one of its acid addition salts by treatment with a base.

3. Use of compounds of the formula I according to Claim 1 as components in liquid-crystal dielectrics for electrooptical display elements.

4. Liquid-crystal dielectric for electrooptical display elements having at least two liquid-crystal components, characterised in that at least one component is a compound of the formula I according to Claim 1.

5. Electrooptical display element, characterised in that it contains a dielectric according to Claim 4.

## Revendications

1. Dérivés de tétrahydropyranne de formule I

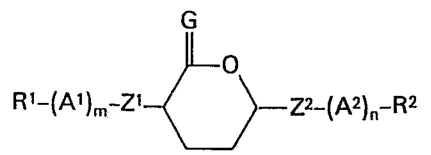

I

où

G représente $H_2$ ou =O,

$R^1$ et $R^2$ représentent chacun un groupe alcoyle en $C_1$ à $C_{10}$, où également un ou deux groupes $CH_2$ peuvent être remplacés par des atomes de O, F, Cl, Br ou CN,

$R^2$ représente également H,

$A^1$ et $A^2$ représentent chacun des groupes 1,4-phénylène, 1,4-cyclohexylène, 1,3-dioxan-2,5-diyle, pipéridin-1,4-diyle, 1,4-bicyclo[2,2,2]-octylène ou pyrimidin-2,5-diyle, non substitués ou substitués par de 1 à 4 atomes de F,

$Z^1$ et $Z^2$ représentent chacun -CO-O-, -O-CO-, $CH_2-CH_2-$ $-OCH_2$, $-CH_2O-$ ou une liaison simple,

m et n représentent chacun 0, 1, 2 ou 3,

(m + n) vaut cependant au moins 1 et au plus 3, où pour m = 2 ou 3 les groupes $A^1$ et pour n = 2 ou 3 les groupes $A^2$ peuvent être chacun identiques ou différents l'un de l'autre,

ainsi que les sels d'addition d'acide des composés basiques parmi ces composés.

2. Procédé de préparation de tétrahydropyrannes de formule I selon la revendication 1, caractérisé en ce qu'on traite avec un agent réducteur un composé, qui sinon correspond à la formule I, mais contient à la place d'atomes de H un ou plusieurs groupes réductibles et/ou liaisons C–C,

ou en ce qu'on cyclise un composé de formule II

$R^1-(A^1)_m-Z^1-CH(CGOH)-CH_2CH_2-CHOH-Z^2-(A^2)_n-R^2$ II

où
G, $R^1$, $R^2$, $A^1$, $A^2$, $Z^1$, $Z^2$, m et n ont les significations indiquées,
ou un de ses dérivés réactifs,
ou en ce qu'on décarboxyle un composé de formule III

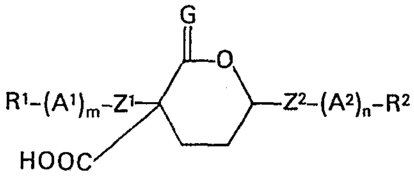

III

où
G, $R^1$, $R^2$, $A^1$, $A^2$, $Z^1$, $Z^2$, m et n
ont les significations indiquées,
ou en ce que pour préparer des composés de formule I, où $R^1$ et/ou $R^2$ représentent F, Cl, Br ou CN, on remplace dans un sel de diazonium correspondant le groupe diazonium par F, Cl, Br ou CN,
ou en ce que pour préparer des esters de formule I (où $Z^1$ et/ou $Z^2$ représentent -CO-O- ou -O-CO-) on fait réagir un acide carboxylique correspondant ou un de ses dérivés réactifs avec un alcool correspondant ou un de ses dérivés réactifs,
ou en ce que pour préparer des dérivés de dioxanne de formule I (où $A^1$ et/ou $A^2$ représentent 1,3-dioxan-2,5-diyle) on fait réagir un aldéhyde correspondant avec un diol correspondant,
ou en ce que pour préparer des nitriles de formule I (où $R^1$ et/ou $R^2$ représentent CN) on déshydrate un amide d'acide carboxylique correspondant ou on fait réagir un halogénure d'acide carboxylique correspondant avec un sulfamide,
ou en ce que pour préparer des éthers de formule I (où $R^1$ et/ou $R^2$ représentent des chaînes alcoyles, où un ou deux groupes $CH_2$ sont remplacés par des atomes de O, et/ou $Z^1$ et/ou $Z^2$ sont des groupes $-OCH_2-$ ou $-CH_2O-$) on éthérifie un composé hydroxy correspondant, et/ou en ce que le cas échéant on fait réagir un composé de chlore ou de brome de formule I (où $R^1$ et/ou $R^2$ représentent Cl ou Br) avec un cyanure,
et/ou en ce que le cas échéant on transforme une base de formule I par traitement avec un acide en un de ses sels d'addition d'acide,
ou en ce que le cas échéant on libère un composé de formule I à partir d'un de ses sels d'addition d'acide pour traitement avec une base.

3. Application des composés de formule I selon la revendication I comme composants de diélectriques à cristaux liquides pour éléments d'affichage électro-optiques.

4. Diélectrique à cristaux liquides pour éléments d'affichage électro-optiques avec au moins deux composants à cristaux liquides, caractérisé en ce qu'au moins un composant est un composé de formule I selon la revendication 1.

5. Elément d'affichage électro-optique, caractérisé en ce qu'il contient un diélectrique selon la revendication 4.